# EUROPEAN PATENT APPLICATION

(11) **EP 3 943 081 A1**
(43) Date of publication of application: **26.01.2022**
(21) Application number: 21180245.9
(22) Date of filing: 12.03.2015
(51) Int. Cl.: A61K 31/202, A01N 25/26, A61P 27/06, A61P 27/12, A61P 39/06

(54) **OPTIC NEUROPATHY TREATMENT AND REDUCTION OF STEROID- INDUCED OXIDATIVE STRESS WITH STABILIZED POLYUNSATURATED SUBSTANCES**

(30) Priority: 13.03.2014 US 201461952419 P
(62) Divisional of application: 15761233.4
(71) Applicant: Retrotope, Inc., Los Altos, CA 94022 (US)
(72) Inventor: SHCHEPINOV, Mikhail S., Los Altos Hills, 94022-3319 (US)
(74) Representative: Gibson, Mark

(57) **Abstract**

Some aspects of the invention provide for a method of treating a patient having glaucoma, a non-hereditary optic neuropathy, steroid-induced oxidative stress, or steroid-induced oxidation-related disorders, comprising identifying a patient having a non-hereditary optic neuropathy or steroid-induced oxidative stress or steroid-induced oxidation-related disorders and in need of treatment; repeatedly administering a polyunsaturated substance to the patient or the patient's tissue, wherein the polyunsaturated substance is chemically modified such that one or more bonds is stabilized against oxidation; wherein following said administration, the polyunsaturated substance or a polyunsaturated metabolite thereof comprising said one or more chemical modifications is incorporated into the patient's body or the patient's tissue.

## Description

### BACKGROUND

### Field

Isotopically modified polyunsaturated fatty acids ("PUFAs") and other modified PUFAs for stabilizing polyunsaturated substances in a patient, particularly for patients with oxidation-related disorders such as glaucoma, a non-hereditary optic neuropathy, steroid-induced oxidative stress, or a steroid-induced oxidation-related disorders.

### Description of the Related Art

The number of diseases associated with oxidative stress are numerous and diverse, but it is well established that oxidative stress is caused by disturbances to the normal redox state within cells. An imbalance between routine production and detoxification of reactive oxygen species ("ROS") such as peroxides and free radicals can result in oxidative damage to cellular structures and machinery. Under normal conditions, potentially important sources of ROSs in aerobic organisms is the leakage of activated oxygen from mitochondria during normal oxidative respiration. Additionally, it is known that macrophages and enzymatic reactions also contribute to the generation of ROSs within cells. Because cells and their internal organelles are lipid membrane-bound, ROSs can readily contact membrane constituents and cause lipid oxidation. Ultimately, such oxidative damage can be relayed to other biomolecules within the cell, such as DNA and proteins, through direct and indirect contact with activated oxygen, oxidized membrane constituents, or other oxidized cellular components. Thus, one can readily envision how oxidative damage may propagate throughout a cell given the mobility of internal constituents and the interconnectedness of cellular pathways.

Lipid-forming fatty acids are well-known as one of the major components of living cells. As such, they participate in numerous metabolic pathways, and play an important role in a variety of pathologies. Polyunsaturated Fatty Acids ("PUFAs") are an important sub¬class of fatty acids. An essential nutrient is a food component that directly, or via conversion, serves an essential biological function and which is not produced endogenously or in large enough amounts to cover the requirements. For homeothermic animals, the two rigorously essential PUFAs are linoleic *(cis,* cis-9,12-Octadecadienoic acid; (9Z,12Z)-9,12-Octadecadienoic acid; "LA"; 18:2;n-6) and alpha-linolenic (*cis*,*cis*,*cis*-9,12,15-Octadecatrienoic acid; (9Z,12Z,15Z)-9,12,15-Octadecatrienoic acid; "ALA"; 18:3;n-3) acids, formerly known as vitamin F (Cunnane SC. Progress in Lipid Research 2003; 42:544-568). LA, by further enzymatic desaturation and elongation, is converted into higher n-6 PUFAs such as arachidonic (AA; 20:4;n-6) acid; whereas ALA gives rise to a higher n-3 series, including, but not limited to, eicosapentaenoic acid (EPA; 20:5;n-3) and docosahexaenoic (DHA; 22:6;n-3) acid (Goyens PL. et al. Am. J. Clin. Nutr. 2006; 84:44-53). Because of the essential nature of certain PUFAs or PUFA precursors, there are many known instances of their deficiency and these are often linked to medical conditions. Furthermore, many PUFA supplements are available over-the-counter, with proven efficiency against certain ailments (See, for example, U.S. Patent No.: 7,271,315 and U.S. Patent No.: 7,381,558).

PUFAs endow mitochondrial membranes with appropriate fluidity necessary for optimal oxidative phosphorylation performance. PUFAs also play an important role in initiation and propagation of the oxidative stress. PUFAs react with ROS through a chain reaction that amplifies an original event (Sun M, Salomon RG, J. Am. Chem. Soc. 2004; 126: 5699-5708). However, non-enzymatic formation of high levels of lipid hydroperoxides is known to result in several detrimental changes. Indeed, Coenzyme Q10 has been linked to increased PUFA toxicity via PUFA peroxidation and the toxicity of the resulting products (Do TQ et al, PNAS USA 1996; 93:7534-7539). Such oxidized products negatively affect the fluidity and permeability of their membranes; they lead to oxidation of membrane proteins; and they can be converted into a large number of highly reactive carbonyl compounds. The latter include reactive species such as acrolein, malonic dialdehyde, glyoxal, methylglyoxal, etc. (Negre-Salvayre A, et al. Brit. J. Pharmacol. 2008; 153:6-20). But the most prominent products of PUFA oxidation are alpha, betaunsaturated aldehydes such as 4-hydroxynon-2-enal (4-HNE; formed from n-6 PUFAs like LA or AA), 4-hydroxyhex-2-enal (4-HHE; formed from n-3 PUFAs like ALA or DHA), and corresponding ketoaldehydes (Esterfbauer H, et al. Free Rad. Biol. Med. 1991; 11:81-128; Long EK, Picklo MJ. Free Rad. Biol. Med. 2010; 49:1-8). These reactive carbonyls cross-link (bio)molecules through Michael addition or Schiff base formation pathways, and have been implicated in a large number of pathological processes (such as those introduced above), agerelated and oxidative stress-related conditions, and aging. Importantly, in some cases, PUFAs appear to oxidize at specific sites because methylene groups of 1,4-diene systems (the bis-allylic position) are substantially less stable to ROS, and to enzymes such as cyclogenases and lipoxygenases, than allylic methylenes.

We have now discovered oxidation resistant PUFAs, PUFA mimetics, PUFA pro-drugs and/or fats containing oxidation resistant PUFAs and PUFA mimetics that are useful for stabilizing polyunsaturated substances in patients, particularly in patients having oxidation-related disorders or subjected to undesirable oxidative stress.

### SUMMARY

Some embodiments provide a method of treating glaucoma, a non-hereditary optic neuropathy, steroid-induced oxidative stress, or a steroid-induced oxidation-related disorder, comprising identifying a patient having glaucoma, a non-hereditary optic neuropathy, steroid-induced oxidative stress, or a steroid-induced oxidation-related disorders and in need of treatment; repeatedly administering a polyunsaturated substance to the patient or the patient's tissue, wherein the polyunsaturated substance is chemically modified such that one or more bonds is stabilized against oxidation; wherein following said administration, the polyunsaturated substance or a polyunsaturated metabolite thereof comprising said one or more chemical modifications is incorporated into the patient's body or the patient's tissue.

In some embodiments, the patient is classified as having a non-hereditary optic neuropathy. In some embodiments, the polyunsaturated substance is repeatedly administered to the patient beginning prior to surgical intervention or the administration of a second therapeutic to treat the optic neuropathy. In some embodiments, the polyunsaturated substance is repeatedly administered to the patient beginning concurrently with surgical intervention or the administration of a second therapeutic to treat the optic neuropathy. In some embodiments, the polyunsaturated substance is repeatedly administered to the patient beginning after surgical intervention or the administration of a second therapeutic to treat the optic neuropathy.

In some embodiments, the patient is classified as having glaucoma. In some embodiments, the non-hereditary optic neuropathy is glaucoma. In some embodiments, the glaucoma is primary glaucoma, developmental glaucoma, secondary glaucoma, or absolute glaucoma. In some embodiments, the glaucoma is primary angle closure glaucoma, primary open-angle glaucoma, pigmentary glaucoma, or exfoliation glaucoma. In some embodiments, the glaucoma is primary congenital glaucoma, infantile glaucoma, or inheritable glaucoma. In some embodiments, the glaucoma is an inflammatory glaucoma, a phacogenic glaucoma, a glaucoma secondary to intraocular hemorrhage, a traumatic glaucoma, a neovascular glaucoma, a drug-induced glaucoma, or a glaucoma of miscellaneous origin.

In some embodiments, the patient is classified as having steroid-induced oxidative stress. In some embodiments, the polyunsaturated substance is repeatedly administered to the patient beginning prior to the administration of a steroid for treatment of a disease or symptom. In some embodiments, the polyunsaturated substance is repeatedly administered to the patient beginning concurrently with the administration of a steroid for treatment of a disease or symptom. In some embodiments, the polyunsaturated substance is repeatedly administered to the patient beginning after the administration of a steroid for treatment of a disease or symptom. In some embodiments, the patient is classified as having a steroid-induced oxidation-related disorder. In some embodiments, the polyunsaturated substance treats, reduces, or inhibits cataracts or cataract formation. In some embodiments, the steroid-induced oxidation disorder is a cataract.

In some embodiments, the patient is a mammal. In other embodiments, the patient is a human. In other embodiments, the patient is a male. In other embodiments, the patient is a female.

In some embodiments, the polyunsaturated substance is a fatty acid, a fatty acid mimetic, or a fatty acid pro-drug. In some embodiments, the fatty acid, fatty acid mimetic, or fatty acid pro-drug is stabilized at one or more bis-allylic positions. In some embodiments, the stabilization comprises at least one ¹³C atom or at least one deuterium atom at a bis-allylic position, wherein the at least one ¹³C atom or the at least one deuterium atom is present at a level significantly above the naturally-occurring abundance level of said isotope. In some embodiments, the fatty acid pro-drug is an ester. In some embodiments, the ester is a triglyceride, diglyceride, or monoglyceride. In some embodiments, the polyunsaturated substance is a fatty acid pro-drug ester and the ester is an alkyl ester. In other embodiments, the polyunsaturated substance is a fatty acid pro-drug ester and the ester is an ethyl ester.

In some embodiments, the stabilized fatty acid, fatty acid mimetic, or fatty acid pro-drug comprise between about 10% and 50% of the total amount of fatty acids, fatty acid mimetics, or fatty acid pro-drugs administered to the patient. In other embodiments, the stabilized fatty acid, fatty acid mimetic, or fatty acid pro-drug comprise between about 10% and 30% of the total amount of fatty acids, fatty acid mimetics, or fatty acid pro-drugs administered to the patient. In other embodiments, the stabilized fatty acid, fatty acid mimetic, or fatty acid pro-drug comprise about 20% or more of the total amount of fatty acids, fatty acid mimetics, or fatty acid pro-drugs administered to the patient. In some embodiments, the stabilized fatty acid, fatty acid mimetic, or fatty acid pro-drug is isotopically stabilized.

In some embodiments, a cell or tissue of the patient maintains a sufficient concentration of the fatty acid, fatty acid mimetic, or fatty acid pro-drug to prevent autooxidation of the naturally occurring polyunsaturated fatty acid, mimetic, or ester pro-drug.

In some embodiments, the polyunsaturated substance is an omega-3 fatty acid, fatty acid mimetic, or fatty acid pro-drug, or an omega-6 fatty acid, fatty acid mimetic, or fatty acid pro-drug. In some embodiments, the polyunsaturated substance is selected from the group consisting of 11,11-D2-linolenic acid, 14,14-D2-linolenic acid, 11,11,14,14-D4-linolenic acid, 11,11-D2-linoleic acid, 14,14-D2-linoleic acid, 11,11,14,14-D4-linoleic acid, 11 -D-linolenic acid, 14-D-linolenic acid, 11,14-D2-linolenic acid, 11-D-linoleic acid, 14-D-linoleic acid, and 11,14-D2-linoleic acid. In some embodiments, the polyunsaturated substance is selected from the group consisting of 9,10,12,13,15,16-D6-linolenic acid, 9,10,11,12,13,15,16-D7-linolenic acid, 9,10,11,11,12,13,15,16-D8-linolenic acid, 9,10,12,13,14,14,15,16-D8-linolenic acid, 9,10,12,13,14,15,16-D7-linolenic acid, 9,10,11,11,12,13,14,14,15,16-D10-linolenic acid, 9,10,11,12,13,14,15,16-D8-linolenic acid, 9,10,12,13-D4-linoleic acid, 9,10,11,12,13-D5-linoleic acid, 9,10,11,11,12,13-D6-linoleic acid, or alkyl ester pro-drug thereof In some embodiments, the polyunsaturated substance is 9,10,12,13-D4-linoleic acid or an alkyl ester pro-drug thereof. In some embodiments, the polyunsaturated substance is further stabilized at a pro-bis-allylic position.

In other embodiments, the polyunsaturated substance is further chemically modified at one or more bis-allylic positions. In some embodiments, the polyunsaturated substance is further chemically modified at one or more bis-allylic positions, wherein the chemical modification comprises at least one ¹³C atom at the one or more bis-allylic positions or at least one deuterium atom at the one or more bis-allylic positions, and wherein the at least one ¹³C atom or the at least one deuterium atom is present at a level significantly above the naturally-occurring abundance level of said isotope. In some embodiments, the polyunsaturated substance is further chemically modified at one or more bis-allylic positions, wherein the chemical modification comprises at least one or more deuterium atoms at the one or more bis-allylic positions, and wherein the at least one or more deuterium atoms is present at a level significantly above the naturally-occurring abundance level of said isotope. In other embodiments, the polyunsaturated substance is further chemically modified at one or more bis-allylic positions, wherein the chemical modification comprises at two deuterium atoms at one or more bis-allylic positions, and wherein the two deuterium atoms are present at a level significantly above the naturally-occurring abundance level of said isotope.

In some embodiments, the polyunsaturated substance is chemically modified such that formation of a radical at an allylic or bis-allylic position of the polyunsaturated substance results in the radical being delocalized across at least two carbon atoms, wherein at least one of the carbon atoms is ¹³C or ¹²C covalently bonded to deuterium.

In some embodiments, all of the carbon atoms in the polyunsaturated substance are not isotopically modified or all of the hydrogen atoms in the polyunsaturated substance are not isotopically substituted.

In some embodiments, an antioxidant is co-administered. Such antioxidants include, but are not limited to Coenzyme Q, idebenone, mitoquinone, mitoquinol, vitamin C, or vitamin E. In some embodiments, the antioxidant is Coenzyme Q, idebenone, mitoquinone, or mitoquinol. In other embodiments, the antioxidant is a mitochondrially-targeted antioxidant. In some embodiments, the antioxidant is a vitamin, vitamin mimetic, or vitamin pro-drug. In other embodiments, the antioxidant is a vitamin E, vitamin E mimetic, vitamin E pro-drug, vitamin C, vitamin C mimetic, and/or vitamin C pro-drug.

### DETAILED DESCRIPTION OF THE PREFERRED EMBODIMENT

### Definitions:

As used herein, abbreviations are defined as follows:
- 4-HHE or HHE: 4-Hydroxyhex-2-enal
- 4-HNE or HNE: 4-Hydroxynon-2-enal
- AA: Arachidonic acid
- ALA: Alpha-linolenic acid
- D: Deuterated
- D1: Mono-deuterated
- D2: Di-deuterated
- D2-LA: Di-deuterated linoleic acid
- D3: Tri-deuterated
- D4: Tetra-deuterated
- D5: Penta-deuterated
- D6: Hexa-deuterated
- DHA: Docosahexaenoic (22:6; n-3) acid
- EPA: Eicosapentaenoic (20:5; n-3) acid
- H-PUFA: Non-deuterated polyunsaturated fatty acid
- LA: Linoleic acid
- PUFA(s): Polyunsaturated fatty acid(s)
- ROS: Reactive oxygen species
- SNOMED: Systematized Nomenclature of Medicine
- TDMS: Toxicology Data Management System
- WT: Wild type
- YPD: Medium containing 1% Bacto-yeast extract, 2% Bacto-peptone, 2% dextrose

Unless otherwise defined, all technical and scientific terms used herein have the same meaning as commonly understood by one of ordinary skill in the art to which these embodiments belong. The terminology used in the description herein is for describing particular embodiments only and is not intended to be limiting of the embodiments. As used in the specification and the appended claims, the singular forms "a," "an," and "the" are intended to include the plural forms as well, unless the context clearly indicates otherwise. All publications, patent applications, patents, and other references mentioned herein are incorporated by reference in their entirety.

Terms and phrases used in this application, and variations thereof, especially in the appended claims, unless otherwise expressly stated, should be construed as open ended as opposed to limiting. As examples of the foregoing, the term 'including' should be read to mean 'including, without limitation,' including but not limited to,' or the like; the term 'comprising' as used herein is synonymous with 'including,' containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide exemplary instances of the item in discussion, not an exhaustive or limiting list thereof; and use of terms like 'preferably,' preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular embodiment. In addition, the term "comprising" is to be interpreted synonymously with the phrases "having at least" or "including at least". When used in the context of a process, the term "comprising" means that the process includes at least the recited steps, but may include additional steps. When used in the context of a compound, composition or device, the term "comprising" means that the compound, composition or device includes at least the recited features or components, but may also include additional features or components. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

It is understood that, in any compound described herein having one or more chiral centers, if an absolute stereochemistry is not expressly indicated, then each center may independently be of R-configuration or S-configuration or a mixture thereof. Thus, the compounds provided herein may be enantiomerically pure, enantiomerically enriched, racemic mixture, diastereomerically pure, diastereomerically enriched, or a stereoisomeric mixture. In addition it is understood that, in any compound described herein having one or more double bond(s) generating geometrical isomers that can be defined as E or Z, each double bond may independently be E or Z a mixture thereof.

Likewise, it is understood that, in any compound described, all tautomeric forms are also intended to be included.

It is to be understood that where compounds disclosed herein have unfilled valencies, then the valencies are to be filled with hydrogens.

As used herein, "alkyl" refers to a straight or branched hydrocarbon chain that comprises a fully saturated (no double or triple bonds) hydrocarbon group. The alkyl group may have 1 to 25 carbon atoms (whenever it appears herein, a numerical range such as "1 to 25" refers to each integer in the given range; *e.g.,* "1 to 25 carbon atoms" means that the alkyl group may consist of 1 carbon atom, 2 carbon atoms, 3 carbon atoms, etc., up to and including 25 carbon atoms, although the present definition also covers the occurrence of the term "alkyl" where no numerical range is designated). The alkyl group may also be a medium size alkyl having 1 to 15 carbon atoms. The alkyl group could also be a lower alkyl having 1 to 6 carbon atoms. The alkyl group of the compounds may be designated as "C₄" or "C₁-C₄ alkyl" or similar designations. By way of example only, "C₁ -C₄ alkyl" indicates that there are one to four carbon atoms in the alkyl chain, i.e., the alkyl chain is selected from methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl and hexyl. The alkyl group may be substituted or unsubstituted.

As used herein, the terms "bis-allylic" or "bis-allylic position" generally refers to the position of a polyunsaturated substance, e.g. a polyunsaturated fatty acid or mimetic thereof, that corresponds to the methylene groups of a 1,4-diene system.

As used herein, the terms "pro-bis-allylic" or "pro-bis-allylic position" refers to the methylene group that becomes the bis-allylic position upon enzymatic desaturation.

As used herein, the term "PUFA" refers to a polyunsaturated fatty acid. Unless otherwise specified, the term "PUFA" also includes esters of the fatty acids. Accordingly, the term "PUFA" includes pharmaceutically acceptable polyunsaturated fatty acid esters.

As used herein, the term "oxidation-related disorder" refers to disorders or symptoms of disorders caused by an imbalance between routine production and detoxification of reactive oxygen species ("ROS"), such as peroxides and free radicals, resulting in oxidative damage to cellular structures.

As used herein, the term "oxidative stress" refers to an imbalance between routine production and detoxification of reactive oxygen species ("ROS"), such as peroxides and free radicals, resulting in oxidative damage to cellular structures. Accordingly, "oxidative stress" leads to "oxidation-related disorders."

As used herein, the term "concurrently" means within a few hours.

As used herein, the term "autoxidation" refers to autocatalytic oxidation, e.g. autocatalytic lipid peroxidation.

All references cited herein, including but not limited to published and unpublished applications, patents, and literature references, are incorporated herein by reference in their entirety and are hereby made a part of this specification. To the extent publications and patents or patent applications incorporated by reference contradict the disclosure contained in the specification, the specification is intended to supersede and/or take precedence over any such contradictory material.

### Optic Neuropathies:

Optic neuropathy refers to damage to the optic nerve due to any cause. Damage and death of these nerve cells, or neurons, leads to characteristic features of optic neuropathy. The main symptom is loss of vision, with colors appearing subtly washed out in the affected eye. On medical examination, the optic nerve head can be visualized by an ophthalmoscope. A pale disc is characteristic of long-standing optic neuropathy. In many cases, only one eye is affected and patients may not be aware of the loss of color vision until the doctor asks them to cover the healthy eye. Optic neuropathy is often called optic atrophy, to describe the loss of some or most of the fibers of the optic nerve. In medicine, "atrophy" usually means "shrunken but capable of regrowth", so some argue that "optic atrophy" as a pathological term is somewhat misleading, and the term "optic neuropathy" should be used instead. In short, optic atrophy is the end result of any disease that damages nerve cells anywhere between the retinal ganglion cells and the lateral geniculate body (anterior visual system).

Various optic neuropathies have been identified, including both hereditary and non-hereditary variants. As used herein, non-hereditary optic neuropathies are traumatic optic neuropathy; infiltrative optic neuropathy; compressive optic neuropathy; nutritional optic neuropathy; toxic optic neuropathy; optic neuritis; ischemic optic neuropathy; anterior ischemic optic neuropathy (AION); non-arteric AION (NAION); radiation optic neuropathy (RON); posterior ischemic optic neuropathy; and glaucoma. Non-hereditary optic neuropathies are Leber's hereditary optic neuropathy (LHON); dominant optic atrophy; and Behr's syndrome.

ROS mediated DNA damage is implicated in a wide variety of disease states. Evidence now indicates that oxidative stress plays a major role in the development of various optic neuropathies, especially glaucoma. The pathogenic role of ROS in glaucoma is supported by various experimental findings, summarized in Izzoti et al., Mutation Research/Reviews in Mutation Research (2006) 612(2), 105-114. Evidence also suggests that oxidative DNA damage is significantly increased in the ocular epithelium regulating aqueous humor outflow of patients suffering from glaucoma. It is now recognized that increased oxygen levels, exposure to light and high PUFA content lead to increased PUFA peroxidation in the eye tissues. We have previously discovered certain classes of polyunsaturated substances that are particularly well suited for treating, reducing, or inhibiting the effects of oxidative stress and oxidation-related disorders. See, e.g., WO 2007/102030; WO 2011/053870; WO 2012/148926; WO 2012/148930; WO 2012/148927; and WO 2012/148929; the disclosures of which are hereby incorporated by reference in their entirety. Accordingly, some aspects of the present embodiments arise from the discovery that stabilized substances, such as stabilized PUFAs, are useful for treating glaucoma or non-hereditary optic neuropathies. These stabilized substances are particularly useful for treating glaucoma. In some cases, the glaucoma is inheritable. Such stabilized substances are especially useful for administering to patients before, during, or after the development of a non-hereditary optic neuropathy. Moreover, such substances are particularly useful for treating diseases and side effects promoted, facilitated, or caused by conventional treatments of non-hereditary optic neuropathies.

### Steroid Induced Oxidative Stress and Oxidation-related Disorders:

Steroids are taken or administered for a variety of therapeutic uses, including the treatment, suppression, inhibition, or use in allergic diseases; as entiemetics; as antineoplastics; in autoimmune disorders, such as the destruction of erythrocytes; in bronchial asthma; in cerebral edema; in congenital adrenal hyperplasia; in gastrointestinal diseases; in hepatic diseases; in ocular diseases; in organ transplantation; in renal diseases; in replacement therapies; in in inflammation; in rheumatic disorders; in sarcoidosis; in skin diseases; in stroke and spinal cord injury; and in thrombocytosis.

Steroids, however, also result in the formation of cataracts; glaucoma; and myopathy; amongst other toxicities and side effects. *See* Islam et al. Eye (2007) 21, 321-323; Dickerson, Jr. et al. Exp. Eye Res. (1997) 65, 507-516; Haller et al. Arch Opththalmol (2010) 128(3). Indeed, cataracts are a well-established complication of glucocorticoid therapy, related both to dosage and duration of therapy. See Bucala et al. Exp. Eye Res. (1985) 40, 853-863; James, J. Ocular Pharm. & Ther. (2007) 23, 403-420. Children are particularly at risk. Moreover, cessation of steroid therapy may not reverse developed opacities, and the cataracts may progress despite the reduction or cessation of therapy. Standard treatment protocols recommend that patients receiving glucocorticoid therapy receive periodic slit-lamp examination to detect steroid induced cataracts, especially posterior subcapsular cataracts. Studies indicate that multi-functional antioxidants are able to delay cataract formation in animal models. See Randazzo et al. "Orally Active Multi-Functional Antioxidants Delay Cataract Formation in Streptoxotocin (Type 1) Diabetic and Gamma-Irradiated Rats," PLoS One (April 2011) 6(4), e18980. As discussed further below, however, the stochastic nature of antioxidants limits their applicability for treating disorders caused by oxidative stress.

Additionally, myopathy, which is characterized by weakness of proximal limb muscles, is occasionally seen in patients taking large dosages of steroids, especially corticoids, and is also clinically relevant to patients suffering from endogenous Cushing's syndrome. Such myopathy can be of sufficient severity to impair ambulation and is used as an indication for withdrawal of therapy. Moreover, steroid myopathy is also of concern in patients suffering from asthma or chronic obstructive pulmonary disease, with such a complication diminishing respiratory function. Recovery from steroid myopathies may be slow and ultimately incomplete.

As previously mentioned, steroids, and especially glucocorticoids and corticosteroids, have an important role in managing ocular inflammatory diseases. Indeed, after glaucoma filtering surgery, topical steroids are often used to delay the wound-healing process by decreasing fibroblast infiltration, which reduces potential scarring. Steroids, however, are also believed to induce glaucoma, but the pathophysiology of steroid-induced glaucoma is not particularly well-understood. Consequently, a therapeutic regimen that permits the use of steroids after glaucoma filtering surgery, but does not increase the likelihood of glaucoma would be desirable. Similarly, steroid therapy, or hormone therapy, also plays an important role in the treatment and regulation of fertility cycles and fertility disorders. Oxidative stress, however, is a particularly problematic symptom of cause of infertility. *See* Lombardo et al. Asian Journal of Andrology (2011) 13, 690-697; *See* Storey, Int. J. Biol. (2008), 52(5-6), 427-437; Ruder et al, Curr Opin Obstet Gynecol (2009) 21(3), 219-222. Thus, a therapeutic regimen that permits the use of steroids, but does not increase the likelihood of infertility would also be desirable.

Some steroids are known to react with ROS and are especially susceptible to peroxidation reactions. *See* Liu et al., J. Lipid Res. (2013) 54, 244-253. As steroids are important components of lipid membranes, the susceptibility of steroids to oxidative stress leads to increased cellular damage of important membrane components like fatty acids. We have previously discovered certain classes of polyunsaturated substances that are particularly well suited for treating, reducing, or inhibiting the effects of oxidative stress and oxidation-related disorders. See, e.g., WO 2007/102030; WO 2011/053870; WO 2012/148926; WO 2012/148930; WO 2012/148927; and WO 2012/148929; the disclosures of which are hereby incorporated by reference in their entirety. Accordingly, some aspects of the present embodiments arise from the discovery that stabilized substances, such as stabilized PUFAs, are useful for treating oxidative stress and oxidation-related disorders resulting from steroid oxidation. Such stabilized substances are especially useful for administering to patients before, during, or after the administration of steroids. Moreover, such substances are particularly useful for treating diseases and side effects promoted, facilitated, or caused by the administration of steroids.

Smith-Lemli-Opitz syndrome (SLOS) is an example of a steroid-induced oxidation-related disorder caused by mutations in the gene encoding 3 (3-hydroxysterol-A7-reductase and as a result of this defect, 7-dehydrocholesterol (7-DHC) and 8-dehydrocholesterol (8-DHC) accumulate in the fluids and tissues of patients with this syndrome. *See* Liu et al., J. Lipid Res. (2013) 54, 244-253. Both 7- and 8-DHC are susceptible to peroxidation reactions, and several biologically active DHC oxysterols are found in cell and animal models of SLOS. *Id.* Accordingly, some aspects of the present embodiments arise from the discovery that stabilized substances, such as stabilized PUFAs, are useful for treating conditions associated with SLOS, such as increased levels of oxidative stress. Such stabilized substances are especially useful for administering to patients before, during, or after the administration of conventional therapy.

### Compositions and Methods:

Some aspects of the embodiments arise from: (1) an understanding that while polyunsaturated substances such as essential PUFAs are vital for proper functioning of lipid membranes, and in particular for mitochondrial membranes, their inherent drawback, i.e., the propensity to be oxidized by ROS with detrimental outcome, is implicated in a variety of oxidation-related disorders such as glaucoma, non-hereditary optic neuropathies, steroid-induced oxidative stress, and steroid-induced oxidation-related disorders; (2) antioxidants cannot prevent PUFA peroxidation due to stochastic nature of the process and the stability of PUFA peroxidation products (reactive carbonyls) to antioxidant treatment, and (3) the ROS-driven damage of oxidation-prone sites within PUFAs may be overcome by using an approach that makes them less amenable to such oxidations, without compromising any of their beneficial physical properties. Indeed, some aspects of embodiments arise from an understanding that formation of a radical at an allylic or bis-allylic position of a polyunsaturated substance results in the radical being delocalized across at least two carbon atoms. Accordingly and unexpectedly, embodiments possessing stabilized bonds and/or atoms within the radical's delocalization area exhibit enhanced stability to oxidative stress. Some aspects of the embodiments describe the use of the isotope effect to achieve stabilizing effects, only at sites in essential PUFAs and PUFA precursors that matter most for oxidation, while other aspects contemplate other sites in addition to those that matter most for oxidation.

One embodiment provides a method of treating glaucoma, a non-hereditary optic neuropathy, steroid-induced oxidative stress, or a steroid-induced oxidation-related disorder, comprising identifying a patient having glaucoma, a non-hereditary optic neuropathy, steroid-induced oxidative stress, or a steroid-induced oxidation-related disorders and in need of treatment; repeatedly administering a polyunsaturated substance to the patient or the patient's tissue, wherein the polyunsaturated substance is chemically modified such that one or more bonds is stabilized against oxidation; wherein following said administration, the polyunsaturated substance or a polyunsaturated metabolite thereof comprising said one or more chemical modifications is incorporated into the patient's body or the patient's tissue.

In another embodiment, the patient is classified as having a non-hereditary optic neuropathy. In one embodiment, the polyunsaturated substance is repeatedly administered to the patient beginning prior to surgical intervention or the administration of a second therapeutic to treat the optic neuropathy. In one embodiment, the polyunsaturated substance is repeatedly administered to the patient beginning concurrently with surgical intervention or the administration of a second therapeutic to treat the optic neuropathy. In one embodiment, the polyunsaturated substance is repeatedly administered to the patient beginning after surgical intervention or the administration of a second therapeutic to treat the optic neuropathy.

In another embodiment, the patient is classified as having glaucoma. In some embodiments, the non-hereditary optic neuropathy is glaucoma. In some embodiments, the glaucoma is primary glaucoma, developmental glaucoma, secondary glaucoma, or absolute glaucoma. In some embodiments, the glaucoma is primary angle closure glaucoma, primary open-angle glaucoma, pigmentary glaucoma, or exfoliation glaucoma. In some embodiments, the glaucoma is primary congenital glaucoma, infantile glaucoma, or inheritable glaucoma. In some embodiments, the glaucoma is an inflammatory glaucoma, a phacogenic glaucoma, a glaucoma secondary to intraocular hemorrhage, a traumatic glaucoma, a neovascular glaucoma, a drug-induced glaucoma, or a glaucoma of miscellaneous origin.

By way of description, primary glaucoma includes primary angle closure glaucoma, also known as primary closed-angle glaucoma, narrow-angle glaucoma, pupil-block glaucoma, acute congestive glaucoma. Examples are acute angle closure glaucoma; chronic angle closure glaucoma; intermittent angle closure glaucoma; superimposed on chronic open-angle closure glaucoma. Primary glaucoma also includes primary open-angle glaucoma, also known as chronic open-angle glaucoma, chronic simple glaucoma, glaucoma simplex. Examples are hightension glaucoma and low-tension glaucoma. Primary glaucoma also include the variants pigmentary glaucoma and exfoliation glaucoma, also known as pseudoexfoliative glaucoma or glaucoma capsulare. Developmental glaucoma includes primary congenital glaucoma, infantile glaucoma, and glaucoma associated with hereditary familial diseases ("hereditary glaucoma"). Secondary glaucoma includes inflammatory glaucoma, such as uveitis of all types and Fuchs heterochromic iridocyclitis; phacogenic glaucoma, such as angle-closure glaucoma with mature cataract, phacoanaphylactic glaucoma secondary to rupture of lens capsule, phacolytic glaucoma due to phacotoxic meshwork blockage, and subluxation of lens; glaucoma secondary to intraocular hemorrhage, such as hyphema and hemolytic glaucoma, also known as erythroclastic glaucoma; traumatic glaucoma, such as angle recession glaucoma, postsurgical glaucoma, aphakic pupillary block, and ciliary block glaucoma; neovascular glaucoma; drug-induced glaucoma, such as corticosteroid induced glaucoma and alpha-chymotrypsin glaucoma; and glaucoma of miscellaneous origin, such as that associated with intraocular tumors, retinal detachments, glaucoma secondary to severe chemical burns of the eye, that associated with essential iris atrophy, and toxic glaucoma. Absolute glausoma is the end stage of all types of glaucoma, characterized in that the eye has no vision, absence of pupillary light reflex and pupillary response, and has a stony appearance.

In another embodiment, the patient is classified as having steroid-induced oxidative stress. In one embodiment, the polyunsaturated substance is repeatedly administered to the patient beginning prior to the administration of a steroid for treatment of a disease or symptom. In one embodiment, the polyunsaturated substance is repeatedly administered to the patient beginning concurrently with the administration of a steroid for treatment of a disease or symptom. In one embodiment, the polyunsaturated substance is repeatedly administered to the patient beginning after the administration of a steroid for treatment of a disease or symptom. In one embodiment, the patient is classified or further classified as having a steroid-induced oxidation-related disorder.

In another embodiment, the polyunsaturated substance is a fatty acid, a fatty acid mimetic, or a fatty acid pro-drug.

In another embodiment, the fatty acid, fatty acid mimetic, or fatty acid pro-drug is stabilized at one or more bis-allylic positions.

In another embodiment, the stabilization comprises at least one ¹³C atom or at least one deuterium atom at a bis-allylic position, wherein the at least one ¹³C atom or the at least one deuterium atom is present at a level significantly above the naturally-occurring abundance level of said isotope.

In another embodiment, the stabilized fatty acid, fatty acid mimetic, or fatty acid pro-drug comprise between about 10% and 50% of the total amount of fatty acids, fatty acid mimetics, or fatty acid pro-drugs administered to the patient.

In another embodiment, the isotopically stabilized fatty acid, fatty acid mimetic, or fatty acid pro-drug comprise between about 10% and 30% of the total amount of fatty acids, fatty acid mimetics, or fatty acid pro-drugs administered to the patient.

In another embodiment, the isotopically stabilized fatty acid, fatty acid mimetic, or fatty acid pro-drug comprise about 20% or more of the total amount of fatty acids, fatty acid mimetics, or fatty acid pro-drugs administered to the patient.

In another embodiment, a cell or tissue of the patient maintains a sufficient concentration of the fatty acid, fatty acid mimetic, or fatty acid pro-drug to prevent autooxidation of the naturally occurring polyunsaturated fatty acid, mimetic, or ester pro-drug.

In another embodiment, the polyunsaturated substance is an omega-3 fatty acid, fatty acid mimetic, or fatty acid pro-drug, or an omega-6 fatty acid, fatty acid mimetic, or fatty acid pro-drug.

In another embodiment, the polyunsaturated substance is selected from the group consisting of 11,11-D2-linolenic acid, 14,14-D2-linolenic acid, 11,11,14,14-D4-linolenic acid, 11,11-D2-linoleic acid, 14,14-D2-linoleic acid, 11,11,14,14-D4-linoleic acid, 11-D-linolenic acid, 14-D-linolenic acid, 11,14-D2-linolenic acid, 11-D-linoleic acid, 14-D-linoleic acid, and 11,14-D2-linoleic acid.

In another embodiment, the polyunsaturated substance is further stabilized at a pro-bis-allylic position.

In another embodiment, the fatty acid pro-drug is an ester.

In another embodiment, the ester is a triglyceride, diglyceride, or monoglyceride.

In another embodiment, an antioxidant is co-administered.

In another embodiment, the co-administered antioxidant is Coenzyme Q, idebenone, mitoquinone, mitoquinol, vitamin C, or vitamin E

In another embodiment, the polyunsaturated substance is further chemically modified at one or more bis-allylic positions.

In another embodiment, the polyunsaturated substance is selected from the group consisting of 9,10,12,13,15,16-D6-linolenic acid, 9,10,11,12,13,15,16-D7-linolenic acid, 9,10,11,11,12,13,15,16-D8-linolenic acid, 9,10,12,13,14,14,15,16-D8-linolenic acid, 9,10,12,13,14,15,16-D7-linolenic acid, 9,10,11,11,12,13,14,14,15,16-D10-linolenic acid, 9,10,11,12,13,14,15,16-D8-linolenic acid, 9,10,12,13-D4-linoleic acid, 9,10,11,12,13-D5-linoleic acid, 9,10,11,11,12,13-D6-linoleic acid, or alkyl ester pro-drug thereof.

In another embodiment, the polyunsaturated substance is 9,10,12,13-D4-linoleic acid or an alkyl ester pro-drug thereof

In another embodiment, the polyunsaturated substance is further chemically modified at one or more bis-allylic positions, wherein the chemical modification comprises at least one 13C atom at the one or more bis-allylic positions or at least one deuterium atom at the one or more bis-allylic positions, and wherein the at least one 13C atom or the at least one deuterium atom is present at a level significantly above the naturally-occurring abundance level of said isotope.

In another embodiment, the polyunsaturated substance is further chemically modified at one or more bis-allylic positions, wherein the chemical modification comprises at least one or more deuterium atoms at the one or more bis-allylic positions, and wherein the at least one or more deuterium atoms is present at a level significantly above the naturally-occurring abundance level of said isotope.

In another embodiment, the polyunsaturated substance is further chemically modified at one or more bis-allylic positions, and wherein the chemical modification comprises at two deuterium atoms at one or more bis-allylic position, and wherein the two deuterium atoms are present at a level significantly above the naturally-occurring abundance level of said isotope.

In another embodiment, all of the carbon atoms in the polyunsaturated substance are not isotopically modified. In another embodiments, all of the hydrogen atoms in the polyunsaturated substance are not isotopically substituted. In another embodiment, at least one carbon atom is not ¹³C. In another embodiment, at least one hydrogen atom is not deuterium.

In another embodiment, the polyunsaturated substance is 9,10,12,13-D4-linoleic acid or an alkyl ester pro-drug thereof

In some embodiments, an isotopically modified polyunsaturated fatty acid or a mimetic refers to a compound having structural similarity to a naturally occurring PUFA that is stabilized chemically or by reinforcement with one or more isotopes, for example ¹³C and/or deuterium. Generally, if deuterium is used for reinforcement, one or both hydrogens on a methylene group may be reinforced.

In some embodiments, the patient is a mammal (i.e., human, domesticated mammal, horse, dog, cow, etc.). In some embodiments, the mammal is a male. In other embodiments, the mammal is a female. In some embodiments, sperm survival or sperm motility is increased.

In some embodiments, the probability that fertilization of an egg by sperm increases. In some embodiments, the probability increases 5%, 10%, 15%, 20%, 25%, 30%, 35%, 40%, 45%, 50%, 55%, 60%, 65%, 70%, 75%, 80%, 90%, 100%, or a range bounded by any two of the aforementioned numbers, or about any of the aforementioned numbers or ranges. In some embodiments, the probability increases by at least 5%, 10%, 15%, 20%, 25%, 30%, 35%, 40%, 45%, 50%, 55%, 60%, 65%, 70%, 75%, 80%, 90%, 100%, or about any of the aforementioned numbers or ranges.

Some aspects of this invention provide compounds that are analogues of essential PUFAs with one, several, or all bis-allylic positions substituted with heavy isotopes. In some embodiments, the CH₂ groups, which will become the bis-allylic position in a PUFA upon enzymatic conversion, are substituted with one or two heavy isotopes.

In some embodiments, the chemical identity of PUFAs, i.e., the chemical structure without regard to the isotope substitutions or substitutions that mimic isotope substitutions, remains the same upon ingestion. For instance, the chemical identity of essential PUFAs, that is, PUFAs that mammals such as humans do not generally synthesize, may remain identical upon ingestion. In some cases, however, PUFAs may be further extended/desaturated in mammals, thus changing their chemical identity upon ingestion. Similarly with mimetics, the chemical identity may remain unchanged or may be subject to similar extension/desaturation. In some embodiments, PUFAs that are extended, and optionally desaturated, upon ingestion and further metabolism may be referred to as higher homologs.

In some embodiments, naturally-occurring abundance level refers to the level of isotopes, for example ¹³C and/or deuterium that may be incorporated into polyunsaturated substances, such as PUFAs, that would be relative to the natural abundance of the isotope in nature. For example, ¹³C has a natural abundance of roughly 1% ¹³C atoms in total carbon atoms. Thus, the relative percentage of carbon having greater than the natural abundance of ¹³C in the polyunsaturated substances may have greater than the natural abundance level of roughly 1% of its total carbon atoms reinforced with ¹³C, such as 2%, but preferably about 5%, 10%, 15%, 20%, 25%, 30%, 35%, 40%, 45%, 50%, 65%, 60%, 65%, 70%, 75%, 80%, 85%, 90%, 95%, or 100%,%, or a range bounded by any two of the aforementioned numbers, of ¹³C with respect to one or more carbon atoms in each molecule of polyunsaturated substance. In other embodiments, the percentage of total carbon atoms reinforced with ¹³C is at least 5%, 10%, 15%, 20%, 25%, 30%, 35%, 40%, 45%, 50%, 65%, 60%, 65%, 70%, 75%, 80%, 85%, 90%, 95%, or 100%.

Regarding hydrogen, in some embodiments, deuterium has a natural abundance of roughly 0.0156% of all naturally occurring hydrogen in the oceans on earth. Thus, a polyunsaturated substance, such as a PUFA, having greater that the natural abundance of deuterium may have greater than this level or greater than the natural abundance level of roughly 0.0156% of its hydrogen atoms reinforced with deuterium, such as 0.02%, but preferably about 5%, 10%, 15%, 20%, 25%, 30%, 35%, 40%, 45%, 50%, 65%, 60%, 65%, 70%, 75%, 80%, 85%, 90%, 95%, or 100%,%, or a range bounded by any two of the aforementioned numbers, of deuterium with respect to one or more hydrogen atoms in each molecule of polyunsaturated substance. In other embodiments, the percentage of total hydrogen atoms reinforced with deuterium is at least 5%, 10%, 15%, 20%, 25%, 30%, 35%, 40%, 45%, 50%, 65%, 60%, 65%, 70%, 75%, 80%, 85%, 90%, 95%, or 100.

In some aspects, a composition of polyunsaturated substances, such as PUFAs, contains both isotopically modified polyunsaturated substances and isotopically unmodified polyunsaturated substances. The isotopic purity is a comparison between a) the relative number of molecules of isotopically modified polyunsaturated substances, and b) the total molecules of both isotopically modified polyunsaturated substances and polyunsaturated substances with no heavy atoms. In some embodiments, the isotopic purity refers to polyunsaturated substances that are otherwise the same except for the heavy atoms.

In some embodiments, isotopic purity refers to the percentage of molecules of isotopically modified polyunsaturated substances, such as PUFAs, in the composition relative to the total number of molecules of the isotopically modified polyunsaturated substances plus polyunsaturated substances with no heavy atoms. For example, the isotopic purity may be about 5%, 10%, 15%, 20%, 25%, 30%, 35%, 40%, 45%, 50%, 65%, 60%, 65%, 70%, 75%, 80%, 85%, 90%, 95%, or 100%, or a range bounded by any two of the aforementioned numbers. In other embodiments, the isotopic purity is at least 5%, 10%, 15%, 20%, 25%, 30%, 35%, 40%, 45%, 50%, 65%, 60%, 65%, 70%, 75%, 80%, 85%, 90%, 95%, or 100%. In some embodiments, isotopic purity of the polyunsaturated substances may be from about 10%-100%, 10%-95%, 10%-90%, 10%-85%, 10%-80%, 10%-75%, 10%-70%, 10%-65%, 10%-60%, 10%-55%, 10%-50%, 10%-45%, 10%-40%, 10%-35%, 10%-30%, 10%-25%, or 10%-20% of the total number of molecules of the polyunsaturated substances in the composition. In other embodiments, isotopic purity of the polyunsaturated substances may be from about 15%-100%, 15%-95%, 15%-90%, 15%-85%, 15%-80%, 15%-75%, 15%-70%, 15%-65%, 15%-60%, 15%-55%, 15%-50%, 15%-45%, 15%-40%, 15%-35%, 15%-30%, 15%-25%, or 15%-20% of the total number of molecules of the polyunsaturated substances in the composition. In some embodiments, isotopic purity of the polyunsaturated substances may be from about 20%-100%, 20%-95%, 20%-90%, 20%-85%, 20%-80%, 20%-75%, 20%-70%, 20%-65%, 20%-60%, 20%¬55%, 20%-50%, 20%-45%, 20%-40%, 20%-35%, 20%-30%, or 20%-25% of the total number of molecules of the polyunsaturated substances in the composition. Two molecules of an isotopically modified polyunsaturated substance out of a total of 100 total molecules of isotopically modified polyunsaturated substances plus polyunsaturated substances with no heavy atoms will have 2% isotopic purity, regardless of the number of heavy atoms the two isotopically modified molecules contain.

In some aspects, an isotopically modified polyunsaturated substance, such as a PUFA molecule, may contain one deuterium atom, such as when one of the two hydrogens in a methylene group is replaced by deuterium, and thus may be referred to as a "D1" PUFA. Similarly, an isotopically modified PUFA molecule may contain two deuterium atoms, such as when the two hydrogens in a methylene group are both replaced by deuterium, and thus may be referred to as a "D2" PUFA. Similarly, an isotopically modified PUFA molecule may contain three deuterium atoms and may be referred to as a "D3" PUFA. Similarly, an isotopically modified PUFA molecule may contain four deuterium atoms and may be referred to as a "D4" PUFA. In some embodiments, an isotopically modified PUFA molecule may contain five deuterium atoms or six deuterium atoms and may be referred to as a "D5" or "D6" PUFA, respectively.

The number of heavy atoms in a molecule, or the isotopic load, may vary. For example, a molecule with a relatively low isotopic load may contain about 1, 2, 3, 4, 5, or 6 deuterium atoms. A molecule with a moderate isotopic load may contain about 10, 11, 12, 13, 14, 15, 16, 17, 18, 19, or 20 deuterium atoms. In a molecule with a very high load, every hydrogen may be replaced with a deuterium. Thus, the isotopic load refers to the percentage of heavy atoms in each polyunsaturated substance, such as a PUFA molecule. For example, the isotopic load may be about 5%, 10%, 15%, 20%, 25%, 30%, 35%, 40%, 45%, 50%, 65%, 60%, 65%, 70%, 75%, 80%, 85%, 90%, 95%, or 100% of the number of the same type of atoms in comparison to a PUFA with no heavy atoms of the same type (e.g. hydrogen would be the "same type" as deuterium). In some embodiments, the isotopic load is at least 5%, 10%, 15%, 20%, 25%, 30%, 35%, 40%, 45%, 50%, 65%, 60%, 65%, 70%, 75%, 80%, 85%, 90%, 95%, or 100%. Unintended side effects are expected to be reduced where there is high isotopic purity in a PUFA composition but low isotopic load in a given molecule. For example, the metabolic pathways will likely be less affected by using a PUFA composition with high isotopic purity but low isotopic load.

One will readily appreciate that when one of the two hydrogens of a methylene group is replaced with a deuterium atom, the resultant compound may possess a stereocenter. In some embodiments, it may be desirable to use racemic compounds. In other embodiments, it may be desirable to use enantiomerically pure compounds. In additional embodiments, it may be desirable to use diastereomerically pure compounds. In some embodiments, it may be desirable to use mixtures of compounds having enantiomeric excesses and/or diastereomeric excesses of about 5%, 10%, 15%, 20%, 25%, 30%, 35%, 40%, 45%, 50%, 65%, 60%, 65%, 70%, 75%, 80%, 85%, 90%, 95%, or 100%, or a range bounded by any two of the aforementioned numbers. In other embodiments, the enantiomeric excesses and/or diastereomeric excesses is at least 5%, 10%, 15%, 20%, 25%, 30%, 35%, 40%, 45%, 50%, 65%, 60%, 65%, 70%, 75%, 80%, 85%, 90%, 95%, or 100%, or a range bounded by any two of the aforementioned numbers. In some embodiments, it may be preferable to utilize stereochemically pure enantiomers and/or diastereomers of embodiments - such as when contact with chiral molecules is being targeted for attenuating oxidative damage. However, in many circumstances, non-chiral molecules are being targeted for attenuating oxidative damage. In such circumstances, embodiments may be utilized without concern for their stereochemical purity. Moreover, in some embodiments, mixtures of enantiomers and diastereomers may be used even when the compounds are targeting chiral molecules for attenuating oxidative damage.

In some aspects, isotopically modified polyunsaturated substances impart an amount of heavy atoms in a particular tissue. Thus, in some aspects, the amount of heavy molecules will be a particular percentage of the same type of molecules in a tissue. For example, the number of heavy molecules may be about 1%-100% of the total amount of the same type of molecules. In some aspects, 10-50% the molecules are substituted with the same type of heavy molecules.

In some embodiments, a compound with the same chemical bonding structure as an essential PUFA but with a different isotopic composition at particular positions will have significantly and usefully different chemical properties from the unsubstituted compound. The particular positions with respect to oxidation, including oxidation by ROS, comprise bis-allylic positions of essential polyunsaturated fatty acids and their derivatives, as shown below as the compound denoted (1). The essential PUFAs isotopically reinforced at bis-allylic positions shown below will be more stable to the oxidation.

Some embodiments provide for particular methods of using compounds denoted as (2) or salts thereof, whereas the sites can be further reinforced with carbon-13. R¹ = alkyl, H, or cation; m = 1-10; n = 1-5, where at each bis-allylic position, one or both Y atoms are deuterium atoms, for example, 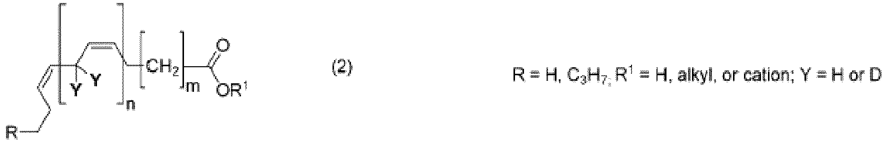 11,11-Dideutero-cis,cis-9,12-Octadecadienoic acid (11,11-Dideutero-(9Z,12Z)-9,12-Octadecadienoic acid; D2-LA); and 11,11,14,14-Tetradeutero-cis,cis,cis-9,12,15-Octadecatrienoic acid (11,11,14,14-Tetradeutero-(9Z,12Z,15Z)-9,12,15-Octadecatrienoic acid; D4-ALA).

In some embodiments, said positions, in addition to deuteration, can be further reinforced by carbon-13, each at levels of isotope abundance above the naturally-occurring abundance level. All other carbon-hydrogen bonds in the PUFA molecule may optionally contain deuterium and/or carbon-13 at, or above, the natural abundance level.

Essential PUFAs are biochemically converted into higher homologues by denaturation and elongation. Therefore, some sites which are not bis-allylic in the precursor PUFAs will become bis-allylic upon biochemical transformation. Such sites then become sensitive to oxidation, including oxidation by ROS. In a further embodiment, such pro-bis-allylic sites, in addition to existing bis-allylic sites are reinforced by isotope substitution as shown below. Accordingly, this aspect of the invention provides for the use of compounds denoted as (3) or salts thereof, where at each bis-allylic position, and at each pro-bis-allylic position, one or more of X or Y atoms may be deuterium atoms. R¹ = alkyl, cation, or H; m = 1¬10; n = 1-5; p = 1-10.

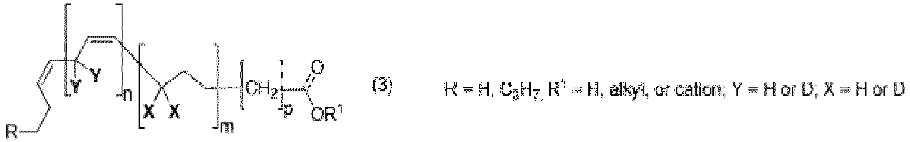

Said positions, in addition to deuteration, can be further reinforced by carbon-13, each at levels of isotope abundance above the naturally-occurring abundance level. In some embodiments, one or more other carbon-hydrogen bonds in the PUFA molecule may independently contain deuterium and/or carbon-13 at or above the natural abundance level.

Oxidation of PUFAs at different bis-allylic sites gives rise to different sets of oxidation products. For example, 4-HNE is formed from n-6 PUFAs whereas 4-HHE is formed from n-3 PUFAs (Negre-Salvayre A, et al. Brit. J. Pharmacol. 2008; 153:6-20). The products of such oxidation possess different regulatory, toxic, signaling, etc. properties. It is therefore desirable to control the relative extent of such oxidations. Accordingly, some aspects of the invention provide for the use of compounds denoted as (6) or salts thereof, differentially reinforced with heavy stable isotopes at selected bis-allylic or pro-bis-allylic positions, to control the relative yield of oxidation at different sites, as shown below, such that any of the pairs of Y¹-Yⁿ and/or X¹ -X^{m} at the bis-allylic or pro-bis-allylic positions of PUFAs may contain deuterium atoms. R¹ = alkyl, cation, or H; m = 1-10; n = 1-6; p = 1-10 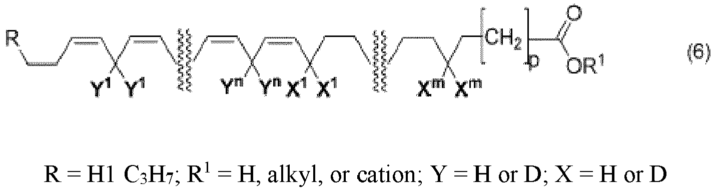 Said positions, in addition to deuteration, can be further reinforced by carbon-13. In some embodiments, one or more other carbon-hydrogen bonds in the PUFA molecule may independently contain deuterium and/or carbon-13 at or above the natural abundance level. It will be appreciated that the break lines in the structure shown above represents a PUFA with a varying number of double bonds, a varying number of total carbons, and a varying combination of isotope reinforced bis-allylic and pro-bis-allylic sites.

Exact structures of compounds illustrated above are shown below that provide for both isotope reinforced n-3 (omega-3) and n-6 (omega-6) essential polyunsaturated fatty acids, and the PUFAs made from them biochemically by desaturation/elongation. Any one of these compounds may be used to slow, inhibit, reduce, or prevent oxidation. In the following compounds, the PUFAs are isotopically reinforced at oxidation sensitive sites and/or sites that may become oxidation sensitive upon biochemical desaturation/elongation. R¹ may be H, alkyl, or cation; R² may be H or D; ^{∗} represents either ¹²C or ¹³C, wherein at least one deuterium or at least one ¹³C is present above the naturally occurring abundance level.

D-Linoleic acids include:

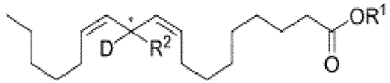

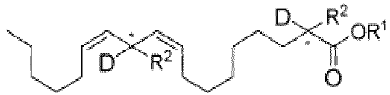

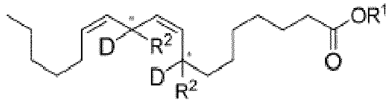

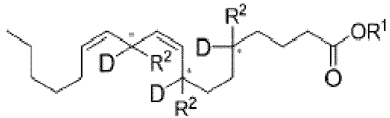

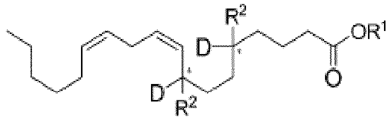

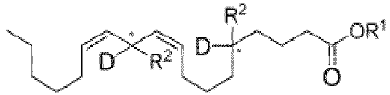

The per-deuterated linoleic acid below may be produced by microbiological methods, for example by growing in media containing deuterium and/or carbon-13.

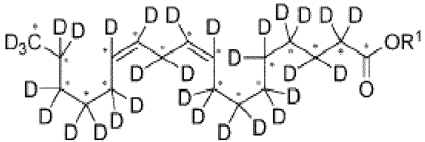

D-Arachidonic acids include:

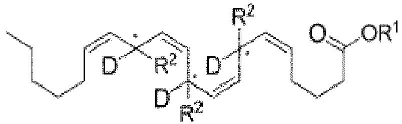

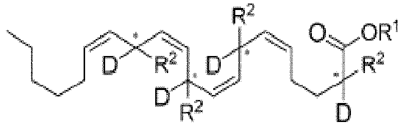

The per-deuterated arachidonic acid below may be produced by microbiological methods, such as by growing in media containing deuterium and/or carbon-13.

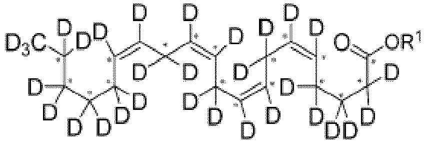

D-Linolenic acids include:

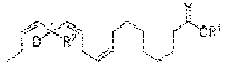

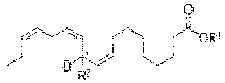

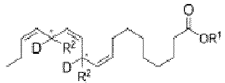

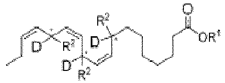

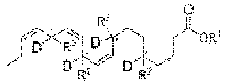

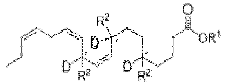

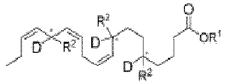

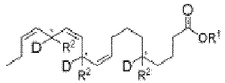

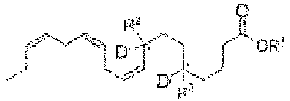

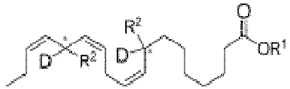

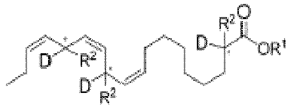

Per-deuterated linolenic acid below may be produced by microbiological methods, such as growing in media containing deuterium and/or carbon-13.

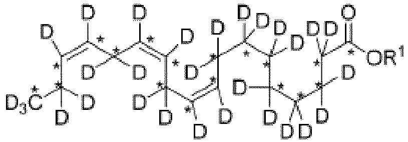

In some aspects of the invention, any PUFAs, whether essential or not, that are capable of being taken up from diet and used in the body, can be utilized. In the case of essential or non-essential PUFAs or precursors, the supplemented stabilized materials can compete with other dietary uptake and bio-manufacture to reduce the available disease-causing species concentrations.

In some aspects of the invention, the polyunsaturated compounds are reinforced at oxidation sensitive positions, as described by way of the structures above, are heavy isotope enriched at said positions as compared to the natural abundance of the appropriate isotope, deuterium and/or carbon-13.

In some embodiments, the disclosed compounds are enriched to 99% isotope purity or more. In some embodiments, the heavy isotope enrichment at said positions is between 50%-99% deuterium and/or carbon-13.

In some embodiments, the modified fatty acids, when dosed via diet as drugs or supplements, may be dosed as pro-drugs, including, but not limited to, non-toxic and pharmaceutically suitable esters of the parent fatty acid or mimetic, such as an ethyl ester or glyceryl ester. This ester assists in tolerance of the drug in the gut, assists in digestion, and relies on the high levels of esterases in the intestines to de-esterify the ester pro-drugs into the active acid form of the drug which adsorbs. Hence, in some embodiments, the invention encompasses the pro-drug esters of the modified fatty acids herein. Examples of this type of drug in the market, nutrition, and clinical trials literature, including Glaxo's Lovaza, (mixtures of omega 3 fatty acid esters, EPA, DHA, and alpha-linolenic acid), Abbott's Omacor (omega-3-fatty acid esters), and most fish oil supplements (DHA and EPA esters). In some aspects, incorporation of the ester pro-drugs into tissues or cells refers to the incorporation of the modified parent PUFA as it would be used as a bodily constituent.

In some embodiments, stabilized compositions mimic natural occurring fatty acids without changing their elemental composition. For example, the substituent may retain the chemical valence shell. Some embodiments include naturally occurring fatty acids, mimetics, and their ester pro-drugs, that are modified chemically to be effective at preventing specific disease mechanisms, but are modified in a way (such as isotopic substitution) that does not change the elemental composition of the material. For example, deuterium is a form of the same element hydrogen. In some aspects, these compounds maintain elemental composition and are stabilized against oxidation. Some compounds that are stabilized against oxidation are stabilized at oxidation sensitive loci. Some compounds are stabilized against oxidation via heavy isotope substitution, then at bis-allylic carbon hydrogen bonds, etc.

In a further embodiment, oxidation-prone bis-allylic sites of PUFAs can be protected against hydrogen abstraction by moving bis-allylic hydrogen-activating double bonds further apart, thus eliminating the bis-allylic positions while retaining certain PUFA fluidity as shown below. These PUFA mimetics have no bis-allylic positions.

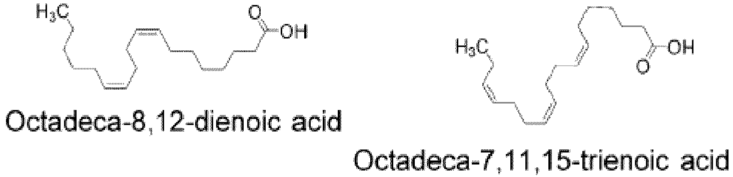

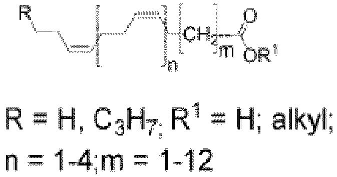

In a further embodiment, oxidation-prone bis-allylic sites of PUFAs can be protected against hydrogen abstraction by using heteroatoms with valence II, thus eliminating the bis-allylic hydrogens as shown below. These PUFA mimetics also have no bis-allylic hydrogens.

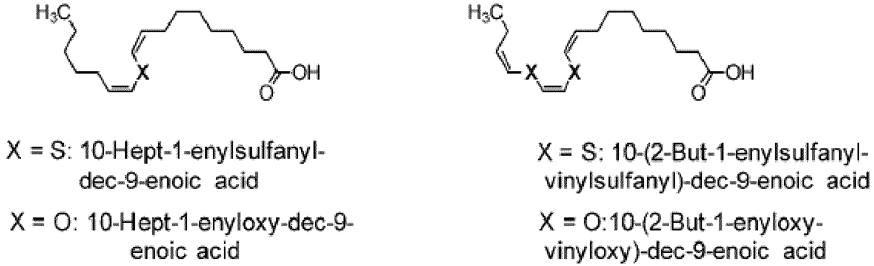

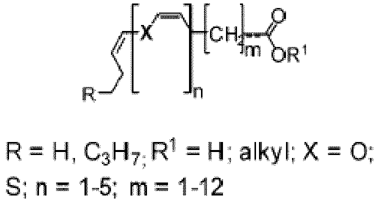

In a further embodiment, PUFA mimetics, i.e. compounds structurally similar to natural PUFAs but unable to get oxidized because of the structural differences, can be employed for the above mentioned purposes. Oxidation-prone bis-allylic sites of PUFAs can be protected against hydrogen abstraction by di-methylation or halogenation as shown below. The hydrogen atoms on the methyl groups may optionally be halogens, such as fluorine, or deuterium. These PUFA mimetics are dimethylated at bis-allylic sites.

In a further embodiment, the PUFA mimetics are dimethylated at bis-allylic sites, represented by the following compounds:

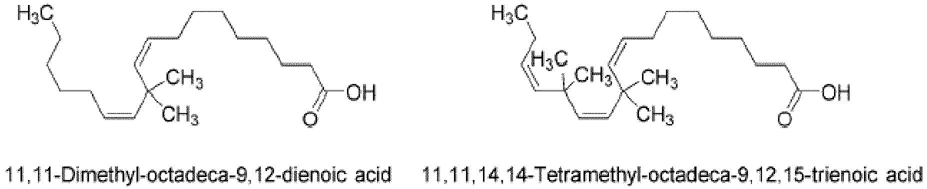

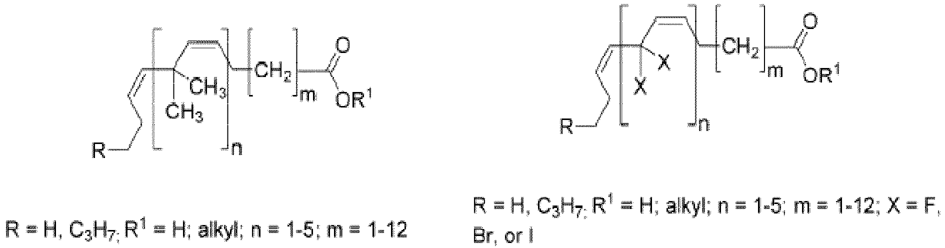

In a further embodiment, oxidation-prone bis-allylic sites of PUFAs can be protected against hydrogen abstraction by alkylation as shown below:

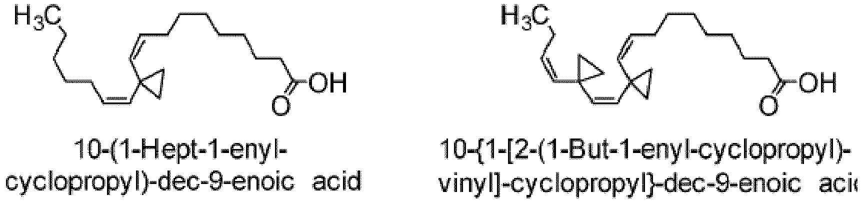

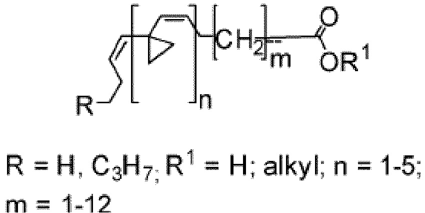

In a further embodiment, cyclopropyl groups can be used instead of double bonds, thus rendering the acids certain fluidity while eliminating the bis-allylic sites as shown below. These PUFA mimetics have cyclopropyl groups instead of double bonds.

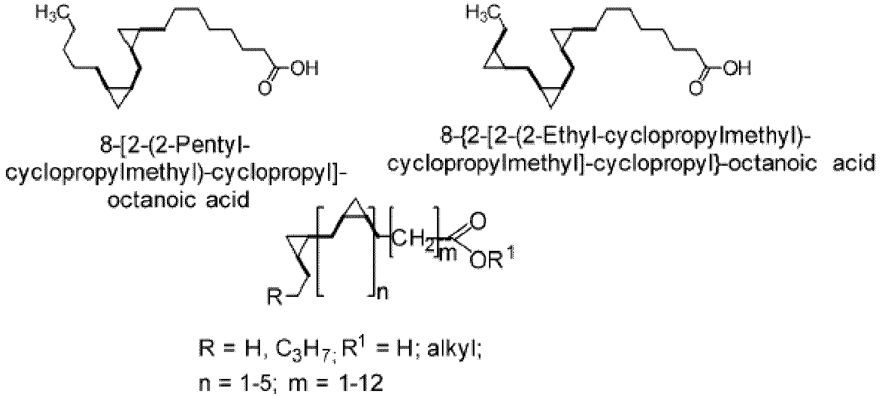

In a further embodiment, 1,2-substituted cyclobutyl groups in appropriate conformation can be used instead of double bonds, thus rendering the acids certain fluidity while eliminating the bis-allylic sites as shown below. These PUFA mimetics have 1,2-cyclobutyl groups instead of double bonds.

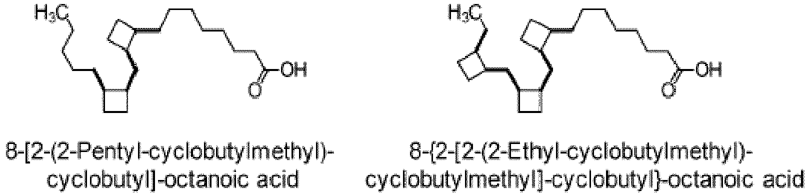

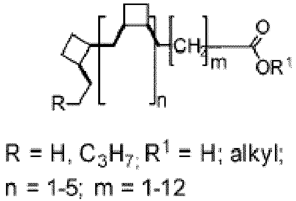

In a modification of the previous embodiment of mimetics with 1,2-cyclobutyl groups instead of double bonds, 1,3-substituted cyclobutyl groups in appropriate conformation can be used instead of double bonds, thus rendering the acids certain fluidity while eliminating the bis-allylic sites. The following PUFA mimetics have 1,3-cyclobutyl groups instead of double bonds.

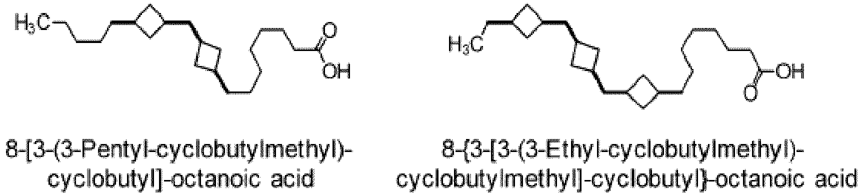

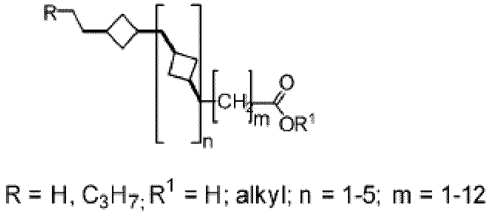

It is a well-known principle in medicinal chemistry that certain functional groups are isosteric and/or bioisosteric with certain other functional groups. Bioisosteres are substituents or groups with similar physical or chemical properties which produce broadly similar biological properties to a chemical compound. For example, isosteres and/or bioisosteres for hydrogen include halogens such as fluorine; isosteres and/or bioisosteres of alkenes include alkynes, phenyl rings, cyclopropyl rings, cyclobutyl rings, cyclopentyl rings, cyclohexyl rings, thioethers, and the like; isosteres and/or bioisosteres of carbonyls include sulfoxides, sulfones, thiocarbonyls, and the like; isosteres and/or bioisosteres of esters include amides, sulfonic acid esters, sulfonamides, sulfinyl acid esters, sulfinylamindes, and the like. Consequently, PUFA mimetics also include compounds having isosteric and/or bioisosteric functional groups.

It is contemplated that it may be useful to formulate PUFAs and/or PUFA mimetics as a pro-drug for use in the invention. A pro-drug is a pharmacological substance may itself have biological activity, but upon administration the pro-drug is metabolized into a form that also exerts biological activity. Many different types of pro-drugs are known and they can be classified into two major types based upon their cellular sites of metabolism. Type I pro-drugs are those that are metabolized intracellularly, while Type II are those that are metabolized extracellularly. It is well-known that carboxylic acids may be converted to esters and various other functional groups to enhance pharmacokinetics such as absorption, distribution, metabolism, and excretion. Esters are a well-known pro-drug form of carboxylic acids formed by the condensation of an alcohol (or its chemical equivalent) with a carboxylic acid (or its chemical equivalent). In some embodiments, alcohols (or their chemical equivalent) for incorporation into pro-drugs of PUFAs include pharmaceutically acceptable alcohols or chemicals that upon metabolism yield pharmaceutically acceptable alcohols. Such alcohols include, but are not limited to, propylene glycol, ethanol, isopropanol, 2-(2-ethoxyethoxy)ethanol (Transcutol^{®}, Gattefosse, Westwood, N.J. 07675), benzyl alcohol, glycerol, polyethylene glycol 200, polyethylene glycol 300, or polyethylene glycol 400; polyoxyethylene castor oil derivatives (for example, polyoxyethyleneglyceroltriricinoleate or polyoxyl 35 castor oil (Cremophor^{®}EL, BASF Corp.), polyoxyethyleneglycerol oxystearate (Cremophor^{®}RH 40 (polyethyleneglycol 40 hydrogenated castor oil) or Cremophor^{®}RH 60 (polyethyleneglycol 60 hydrogenated castor oil), BASF Corp.)); saturated polyglycolized glycerides (for example, Gelucire^{®} 35/10, Gelucire^{®} 44/14, Gelucire^{®} 46/07, Gelucire^{®} 50/13 or Gelucire^{®} 53/10, available from Gattefosse, Westwood, N.J. 07675); polyoxyethylene alkyl ethers (for example, cetomacrogol 1000); polyoxyethylene stearates (for example, PEG-6 stearate, PEG-8 stearate, polyoxyl 40 stearate NF, polyoxyethyl 50 stearate NF, PEG-12 stearate, PEG-20 stearate, PEG-100 stearate, PEG-12 distearate, PEG-32 distearate, or PEG-150 distearate); ethyl oleate, isopropyl palmitate, isopropyl myristate; dimethyl isosorbide; N-methylpyrrolidinone; parafin; cholesterol; lecithin; suppository bases; pharmaceutically acceptable waxes (for example, carnauba wax, yellow wax, white wax, microcrystalline wax, or emulsifying wax); pharmaceutically acceptable silicon fluids; soribitan fatty acid esters (including sorbitan laurate, sorbitan oleate, sorbitan palmitate, or sorbitan stearate); pharmaceutically acceptable saturated fats or pharmaceutically acceptable saturated oils (for example, hydrogenated castor oil (glyceryl-tris-12-hydroxystearate), cetyl esters wax (a mixture of primarily C14-C18 saturated esters of C14-C18 saturated fatty acids having a melting range of about 43°-47° C), or glyceryl monostearate).

In some embodiments, the fatty acid pro-drug is represented by the ester P—B, wherein the radical P is a PUFA and the radical B is a biologically acceptable molecule. Thus, cleavage of the ester P—B affords a PUFA and a biologically acceptable molecule. Such cleavage may be induced by acids, bases, oxidizing agents, and/or reducing agents. Examples of biologically acceptable molecules include, but are not limited to, nutritional materials, peptides, amino acids, proteins, carbohydrates (including mono saccharides, disaccharides, polysaccharides, glycosaminoglycans, and oligosaccharides), nucleotides, nucleosides, lipids (including mono-, diand tri-substituted glycerols, glycerophospholipids, sphingolipids, and steroids).

In some embodiments, alcohols (or their chemical equivalent) for incorporation into pro-drugs of PUFAs include alcohols with 1 to 50 carbon atoms ("C1-50 alcohols"), C₁₋₄₅ alcohols, C₁₋₄₀ alcohols, C₁₋₃₅ alcohols, C₁₋₃₀ alcohols, C₁₋₂₅ alcohols, C₁₋₂₀ alcohols, C₁₋₁₅ alcohols, C₁₋₁₀ alcohols, C₁₋₆ alcohols (whenever it appears herein, a numerical range such as "1-50" refers to each integer in the given range; *e.g.,* "1 to 50 carbon atoms" means that the alkyl group may consist of 1 carbon atom, 2 carbon atoms, 3 carbon atoms, etc., up to and including 50 carbon atoms, although the present definition also covers the occurrence of the term "alkyl" where no numerical range is designated). Such alcohols may be branched, unbranched, saturated, unsaturated, polyunsaturated and/or include one or more heteroatoms such as nitrogen, oxygen, sulfur, phosphorus, boron, silicone, fluorine, chlorine, bromine, or iodine. Exemplary alcohols include methyl, ethyl, propyl, iso-propyl, n-butyl, isobutyl, sec-butyl, tert-butyl, pentyl, hexyl, perfluromethyl, perchloromethyl, perfluoro-tert-butyl, perchloro-tert-butyl, and benzyl alcohols as well as ether alcohols such as polyethylene glycols. In some embodiments, the alcohol contains a charged species. Such species may be anionic or cationic. In some embodiments, the species is a positively charged phosphorus atom. In other embodiments, the positively charged phosphorus atom is a phosphonium cation. In other embodiments the charged species is a primary, secondary, tertiary, or quaternary ammonium cation.

In some embodiments, alcohols (or their chemical equivalent) for incorporation into pro-drugs of PUFAs include polyalcohols such as diols, triols, tetra-ols, pentaols, etc. Examples of polyalcohols include ethylene glycol, propylene glycol, 1,3-butylene glycol, polyethylene glycol, methylpropanediol, ethoxydiglycol, hexylene glycol, dipropylene glycol glycerol, and carbohydrates. Esters formed from polyalcohols and PUFAs may be mono-esters, di-esters, tri-esters, etc. In some embodiments, multiply esterified polyalcohols are esterified with the same PUFAs. In other embodiments, multiply esterified polyalcohols are esterified with different PUFAs. In some embodiments, the different PUFAs are stabilized in the same manner. In other embodiments, the different PUFAs are stabilized in different manners (such as deuterium substitution in one PUFA and ¹³C substitution in another PUFA). In some embodiments, one or more PUFAs are an omega-3 fatty acid and one or more PUFAs are an omega-6 fatty acid.

It is also contemplated that it may be useful to formulate PUFAs and/or PUFA mimetics and/or PUFA pro-drugs as salts for use in the invention. For example, the use of salt formation as a means of tailoring the properties of pharmaceutical compounds is well known. See Stahl et al., Handbook of pharmaceutical salts: Properties, selection and use (2002) Weinheim/Zurich: Wiley-VCH/VHCA; Gould, Salt selection for basic drugs, Int. J. Pharm. (1986), 33:201-217. Salt formation can be used to increase or decrease solubility, to improve stability or toxicity, and to reduce hygroscopicity of a drug product.

Formulation of PUFAs and/or PUFA mimetics and/or PUFA pro-drugs as salts includes, but is not limited to, the use of basic inorganic salt forming agents, basic organic salt forming agents, and salt forming agents containing both acidic and basic functional groups. Various useful inorganic bases for forming salts include, but are not limited to, alkali metal salts such as salts of lithium, sodium, potassium rubidium, cesium, and francium, and alkaline earth metal salts such as berylium, magnesium, calcium, strontium, barium, and radium, and metals such as aluminum. These inorganic bases may further include counterions such as carbonates, hydrogen carbonates, sulfates, hydrogen sulfates, sulfites, hydrogen sulfites, phosphates, hydrogen phosphates, dihydrogen phosphates, phosphites, hydrogen phosphites, hydroxides, oxides, sulfides, alkoxides such as methoxide, ethoxide, and t-butoxide, and the like. Various useful organic bases for forming salts include, but are not limited to, amino acids, basic amino acids such as arginine, lysine, ornithine and the like, ammonia, alkylamines such as methylamine, ethylamine, dimethylamine, diethylamine, trimethylamine, triethylamine and the like, heterocyclic amines such as pyridine, picoline and the like, alkanolamines such as ethanolamine, diethanolamine, triethanolamine and the like, diethylaminoethanol, dimethylaminoethanol, N-methylglucamine, dicyclohexylamine, N,N'-dibenzylethylenediamine, ethylenediamine, piperazine, choline, trolamine, imidazole, diolamine, betaine, tromethamine, meglumine, chloroprocain, procaine, and the like.

Salt formulations of PUFAs and/or PUFA mimetics and/or PUFA pro-drugs include, but are not limited to, pharmaceutically acceptable basic inorganic salts, basic organic salts, and/or organic compounds having both acidic and basic functional groups. Pharmaceutically acceptable salts are well known in the art and include many of the above-recited inorganic and organic bases. Pharmaceutically acceptable salts further include salts and salt-forming agents found in drugs approved by the Food and Drug Administration and foreign regulatory agencies. Pharmaceutically acceptable organic cations for incorporation include, but are not limited to, benzathine, chloroprocaine, choline, diethanolamine, ethylenediamine, meglumine, procaine, benethamine, clemizole, diethylamine, piperazine, and tromethamine. Pharmaceutically acceptable metallic cations for incorporation include, but are not limited to, aluminum, calcium, lithium, magnesium, potassium, sodium, zinc, barium, and bismuth. Additional salt-forming agents include, but are not limited to, arginine, betaine, carnitine, diethylamine, L-glutamine, 2-(4-imidazolyl)ethylamine, isobutanolamine, lysine, N-methylpiperazine, morpholine, and theobromine.

Moreover, several lists of pharmaceutically approved counterions exists. *See* Bighley et α/., Salt forms of drugs and absorption. 1996 In: Swarbrick J. et al. eds. Encyclopaedia of pharmaceutical technology, Vol. 13 New York: Marcel Dekker, Inc. pp 453-499; Gould, P.L., Int. J. Pharm. 1986, 33, 201-217; Berge, J. Pharm. Sci. 1977, 66, 1-19; Heinrich Stahl P., Wermuch C.G. (editors), Handbook of Pharmaceutical Salts, IUPAC, 2002**;** Stahl et al., Handbook of pharmaceutical salts: Properties, selection and use (2002) Weinheim/Zurich: Wiley-VCH/VHCA, all of which are incorporated herein by reference.

Various useful inorganic bases for forming salts include, but are not limited to, alkali metal salts such as salts of lithium, sodium, potassium rubidium, cesium, and francium, and alkaline earth metal salts such as berylium, magnesium, calcium, strontium, barium, and radium, and metals such as aluminum. These inorganic bases may further include counterions such as carbonates, hydrogen carbonates, sulfates, hydrogen sulfates, sulfites, hydrogen sulfites, phosphates, hydrogen phosphates, dihydrogen phosphates, phosphites, hydrogen phosphites, hydroxides, oxides, sulfides, alkoxides such as methoxide, ethoxide, and t-butoxide, and the like.

Various useful organic bases for forming salts include, but are not limited to, amino acids; basic amino acids such as arginine, lysine, ornithine and the like; ammonia; ammonium hydroxide; alkylamines such as methylamine, ethylamine, dimethylamine, diethylamine, trimethylamine, triethylamine and the like; heterocyclic amines such as pyridine, picoline and the like; alkanolamines such as ethanolamine, diethanolamine, triethanolamine and the like, diethylaminoethanol, dimethylaminoethanol; N-methylglucamine; dicyclohexylamine; N,N'-dibenzylethylenediamine; ethylenediamine; piperazine; choline; trolamine; imidazole; diolamine; betaine; tromethamine; meglumine; chloroprocain; procaine; and the like.

In some embodiments, it is unnecessary to substitute all isotopically unmodified PUFAs, such as non-deuterated PUFAs, with isotopically modified PUFAs such as deuterated PUFAs. In some embodiments, is preferable to have sufficient isotopically modified PUFAs such as D-PUFAs in the membrane to prevent unmodified PUFAs such as H-PUFAs from sustaining a chain reaction of self-oxidation. During self-oxidation, when one PUFA oxidizes, and there is a non-oxidized PUFA in the vicinity, the non-oxidized PUFA can get oxidized by the oxidized PUFA. This may also be referred to as autooxidation. In some instances, if there is a low concentration, for example "dilute" H-PUFAs in the membrane with D-PUFAs, this oxidation cycle may be broken due to the distance separating H-PUFAs. In some embodiments, the concentration of isotopically modified PUFAs is present in a sufficient amount to maintain autooxidation chain reaction. To break the autooxidation chain reaction, for example, 1-60%, 5-50%, or 15-35% of the total molecules of the same type are in the membrane. This may be measured by IRMS (isotope ratio mass spectrometry).

A further aspect of the invention provides a dietary, supplementary or pharmaceutical composition of the active compounds. In some embodiments, the dietary, supplementary, or pharmaceutical composition may comprise a salt of the active compound.

### Co-administration

In some embodiments, one or more stabilized substances, such as PUFAs, are administered before, concurrently, or after administration of an additional therapeutic useful to treating or inhibiting certain diseases, or reducing side effects associated with certain diseases. In some embodiments, the PUFA and the additional therapeutic are useful for treating the same disease.

In some embodiments, compounds disclosed herein are administered in combination. For example, in some embodiments, two, three, four, and/or five or more stabilized compounds are administered together. In some embodiments, stabilized compounds are administered in approximately similar amounts. In other embodiments, stabilized compounds are administered in differing amounts. For example, any one of two or more compounds in a mixture may represent about 1% to about 99% of a mixture, about 5% to about 95% of a mixture, about 10% to about 90% of a mixture, about 15% to about 85% of a mixture, about 20% to about 80% of a mixture, about 25% to about 75% of a mixture, about 30% to about 70% of a mixture, about 35% to about 65% of a mixture, about 40% to about 60% of a mixture, about 40% to about 60% of a mixture, about 45% to about 55% of a mixture, and/or about 50% of a mixture. In other embodiments, any one of two or more compounds in a mixture may represent about: 5%, 10%, 15%, 20%, 25%, 30%, 35%, 40%, 45%, 50%, 65%, 60%, 65%, 70%, 75%, 80%, 85%, 90%, 95%, or 100% of a mixture, or a range bounded by any two of the aforementioned numbers.

Although antioxidants cannot cancel the negative effects of PUFA peroxidation due to the stochastic nature of the process and the stability of PUFA peroxidation products (reactive carbonyls) to antioxidant treatment, co-administration of antioxidants with compositions resistant to oxidation, such as those described herein, may prove beneficial for treating oxidative stress-related disorders.

Certain antioxidants contemplated as useful for co-administration include the following: vitamins, such as vitamin C and vitamin E; glutathione, lipoic acid, uric acid, sulforaphane carotenes, lycopene, lutein, anthocyanins, oxalic acid, phytic acid, tannins, coenzyme Q, melatonin, tocopherols, tocotrienols, polyphenols including resveratrol, flavonoids, selenium, eugenol, idebenone, mitoquinone, mitoquinol, ubiquinone, Szeto-Schiller peptides, and mitochondrial-targeted antioxidants. When not explicitly mentioned, quinone derivatives of the aforementioned antioxidants are also contemplated as useful for co-administration.

In some embodiments, stabilized compounds are administered with compounds that upregulate antioxidant genes. In other embodiments, stabilized compounds are administered with compounds that affect signaling pathways, such as the Keap 1 /Nrf2/ARE signaling pathway, thereby resulting in the production of anti-inflammatory and/or antioxidant proteins, such as heme oxygenase-1 (HO-1). In some embodiments, stabilized compounds are administered with antioxidant inflammation modulators. Antioxidant inflammation modulators suppress pro-oxidant and/or pro-inflammatory transcription factors. In some embodiments, antioxidant inflammation modulators are activators of the transcription factor Nrf2. Nrf2 activation promotes the antioxidant, detoxification, and anti-inflammatory genes upregulation. In other embodiments, antioxidant inflammation modulators suppress NF-κB. In some embodiments, antioxidant inflammation modulators suppress STAT3. In other embodiments, stabilized compounds are administered with compounds that affect histone deacetylase activity. In some embodiments, stabilized compounds are administered with compounds that bind to antioxidant response elements (ARE). In other embodiments, stabilized compounds are administered with bardoxolone methyl (2-cyano-3,12-dioxooleane-1,9(11)-dien-28-oic acid methyl ester) as the antioxidant inflammation modulator. In some embodiments, the antioxidant inflammation modulator is 2-cyano-3,12-dioxooleane-1,9(11)-dien-28-oic acid, or a pharmaceutically acceptable ester thereof In other embodiments, the antioxidant inflammation modulator is an amide of 2-cyano-3,12-dioxooleane-1,9(11)-dien-28-oic acid. In some embodiments, the antioxidant inflammation modulator is a triterpenoid. In other embodiments, the antioxidant inflammation modulator is selected from the following compounds:

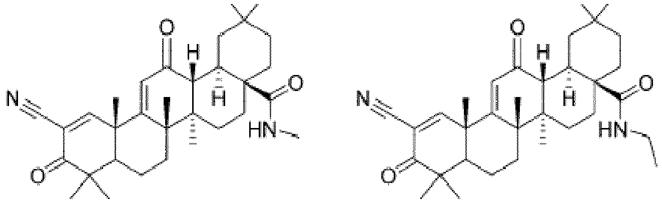

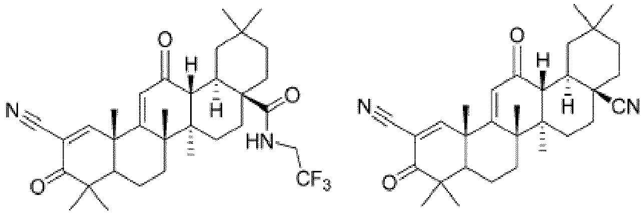

Additional antioxidants believed to be useful in co-administration therapies include those compounds disclosed in U.S. Patent Nos. 6,331,532; 7,179,928; 7,232,809; 7,888,334; 7,888,335; 7,432,305; 7,470,798; and 7,514,461; and U.S. Patent Application Nos. 20020052342; 20030069208; 20040106579; 20050043553; 20050245487; 20060229278; 20070238709; 20070270381; 20080161267; 20080275005; 20090258841; 20100029706; and 20110046219; in which the compounds disclosed therein are incorporated by reference.

Additionally, it is contemplated that co-administration of antioxidants could take the form of consuming foods known to have increased levels of beneficial antioxidants. Such foods include both regular foods and "superfoods" which contain antioxidants. These foods include fruits, vegetables, and other foodstuffs such as strawberries, blackcurrants, blackberries, oranges, blueberries, pomegranates, tea, coffee, olive oil, chocolate, cinnamon, herbs, red wine, grain cereals, eggs, meat, legumes, nuts, spinach, turnip, rhubarb, cocao beans, maize, beans, cabbage, and the like.

In some embodiments, one or more stabilized substances, such as one or more PUFAs, is administered before, concurrently, or after administration of an additional therapeutic useful for treating an ophthalmic disorder, such as an optic neuropathy. In some embodiments, the optic neuropathy is glaucoma. Compounds useful for treating or inhibiting glaucoma, or reducing side effects of glaucoma, are known to one of ordinary skill in the art. Such compounds include cholinergic agonists; anticholinesterase agents; muscarinic antagonists; sympathomimetic agents; a- and 13-andrenergic antagonists; carbonic anhydrase inhibitors; prostaglandin analogs; and marijuana.

In some embodiments, one or more stabilized substances, such as PUFAs, are administered before, concurrently, or after surgical intervention to treat or inhibit an ophthalmic disorder, such as an optic neuropathy. In some embodiments, the optic neuropathy is glaucoma. In some embodiments, the surgical intervention is laser trabeculoplasty; argon laser trabeculoplasty; selective laser trabeculoplasty; iridotomy; laser peripheral iridotomy; iridectomy; sclerotomy; anterior sclerotomy; trabeculectomy; viscocanalostomy; goniotomy; tube-shunt surgery; goniocurretage; cyclodialysis; cyclogoniotomy; canaloplasty; cyclocryotherapy; cyclophotocoagulation; ciliary body ablation; cyclophotoablation; cyclophototherapy; cyclodiathermy; or cycloelectrolysis.

### Delivery and Additional Formulations:

It is well known that triglycerides are the main constituents of vegetable oils and animal fats. It is also known that a triglyceride is an ester compound derived from glycerol and three fatty acids. Triglycerides are metabolized by enzymes such as lipases which hydrolyze ester bonds and release fatty acids and glycerol. Indeed, this metabolism releases fatty acids which can then be taken upon by cells via a fatty acid transporter protein. It is contemplated that PUFAs and PUFA mimetics that are useful in treating various diseases may be incorporated into fats such as triglycerides, diglycerides, and/or monoglycerides for administration to a patient.

The delivery of the PUFAs, PUFA mimetics, PUFA pro-drugs, and triglycerides containing PUFAs and/or PUFA mimetics could be through a modified diet. Alternatively, the PUFAs, PUFA mimetics, PUFA pro-drugs, and triglycerides containing PUFAs and/or PUFA mimetics can be administered as foods or food supplements, on their own or as complexes with 'carriers', including, but not limited to, complexes with albumin.

Other methods of delivering the reinforced PUFAs or their precursors, such as methods typically used for drug delivery and medication delivery, can also be employed. These methods include, but are not limited to, peroral delivery, topical delivery, transmucosal delivery such as nasal delivery, nasal delivery through cribriform plate, intravenous delivery, subcutaneous delivery, inhalation, or through eye drops.

Targeted delivery methods and sustained release methods, including, but not limited to, the liposome delivery method, can also be employed.

It is contemplated that the isotopically modified compounds described herein may be administered over a course of time, in which the cells and tissues of the subject will contain increasing levels of isotopically modified compounds over the course of time in which the compounds are administered. For example, compounds may be administered for 2 days, 3 days, 4 days, 5 days, 6 days, 7 days, 8 days, 10 days, 12 days, 14 days, 16 days, 18 days, 20 days, 25 days, 1 month, 2 months, 3 months, 4 months, five months, 6 months, 9 months, 1 year, 2 years, 5 years, or 10 years, or a range bounded by any two of the aforementioned numbers. In some embodiments, compounds are administered for about 2 months.

Compositions containing the active ingredient may be in a form suitable for oral use, for example, as tablets, troches, lozenges, aqueous or oily suspensions, oil-in-water emulsions, dispersible powders or granules, emulsions, hard or soft capsules, or syrups or elixirs. Such compositions may contain excipients such as bulking agents, solubilization agents, taste masking agents, stabilizers, coloring agents, preservatives and other agents known to those ordinarily skilled in the art of pharmaceutical formulation. In addition, oral forms may include food or food supplements containing the compounds described herein. In some embodiments supplements can be tailor-made so that one type of PUFA, such as omega-3 or omega-6 fatty acids can be added to food or used as a supplement depending on the dominant fat that the food or the subject's diet contains. Moreover, compositions can be tailor-made depending on the disease to be treated. For example, an LDL related condition may require more D-linoleic acid because cardiolipin, which is made of linoleic acid, is oxidized. In other embodiments, such as retinal disease and neurological/CNS conditions may require more omega-3 fatty acids such as D-linolenic acid, because D-omega-3 fatty acids are more relevant for treating these diseases. In some aspects, when the disease is associated with HNE, then D-omega-6 fatty acids should be prescribed, whereas for HHE, D-omega-3 fatty acids should be prescribed.

Compositions may also be suitable for delivery by topical application, as a spray, cream, ointment, lotion, or as a component or additive to a patch, bandage or wound dressing. In addition the compound can be delivered to the site of the disease by mechanical means, or targeted to the site of the disease through the use of systemic targeting technologies such as liposomes (with or without chemical modification that provides them with affinity for the diseased tissue), antibodies, aptamers, lectins, or chemical ligands such as albumin, with affinity for aspects of the diseased tissue that are less abundant or not present on normal tissue. In some aspects, topical application of cosmetics may include the use of a carrier which is an isotopically modified compound or mimetic described herein for delivering through skin such as by a patch. Eye disorders may be treated with eyedrops.

A pharmaceutical composition may also be in a form suitable for administration by injection. Such compositions may be in the form of a solution, a suspension or an emulsion. Such compositions may include stabilizing agents, antimicrobial agents or other materials to improve the function of the medicament. Some aspects of the invention also encompass dry or desiccated forms of the compounds which can readily be formed or reconstituted into a solution suspension or emulsion suitable for administration by injection, or for oral or topical use. Delivery by injection may be suitable for systemic delivery, and also local delivery such as injection into the eye for treating disorders relating to the eye.

### Dosages

In some embodiments, compounds are dosed at about 0.01 mg/kg to about 1000 mg/kg, about 0.1 mg/kg to about 100 mg/kg, and/or about 1 mg/kg to about 10 mg/kg. In other embodiments, compounds are dosed at about: 0.01, 0.1, 1.0, 5.0, 10, 25, 50, 75, 100, 150, 200, 300, 400, 500, and/or 1000 mg/kg. In some embodiments, compounds are dosed by mouth with a morning, afternoon, and/or evening meal. In some embodiments, 0.25 g, 0.5 g, 0.75 g, 1 g, 1.25 g, 1.5 g, 1.75 g, 2 g, 2.5 g, 3 g, 3.5 g, 4 g, 4.5 g, 5 g, 5.5 g, 6 g, 6.5 g, 7 g, 7.5 g, 8 g, 9 g, 10, or 15 g, or a range bounded by any two of the aforementioned numbers are administered daily. In other embodiments, the aforementioned amounts are dosed at a single meal. In some embodiments, the desired daily dose is administered over two or more meals. In some embodiments, 1 g, 2 g, 4 g, or 8 g are administered per day.

### EXAMPLES

Certain compounds useful for the methods disclosed herein can be prepared according to known methodologies, or ready modifications of known methodologies. For example, deuterium substituted cis-9-octadecenoate-14,14,15,15,17,18-d6 and methyl cis-9,cis-12-octadecadienoate-12,13-d2, and triglycerides of these variants can be prepared as previously described in Adlof et al., J. Labelled Compd. Radiopharm. (1978), Vol. 15, pp. 97-104; Adlof et al., J. Am. Oil. Chem. Soc. (1993), Vol. 70, pp. 817-819; and Emken et al., Lipids (1999), Vol. 34, pp. 785-791, the disclosures of which are incorporated herein by reference in their entirety. Some compounds are commercially available.

Additional compounds useful for the methods disclosed herein can be prepared as described in WO 2012/148926; WO 2012/148930; WO 2012/148927; and WO 2012/148929.

One of ordinary skill in the art will recognize that carboxylic acids can be readily converted into additional derivatives, such as esters, by treatment with known reagents and the desired alcohol as described in Scheme 1.

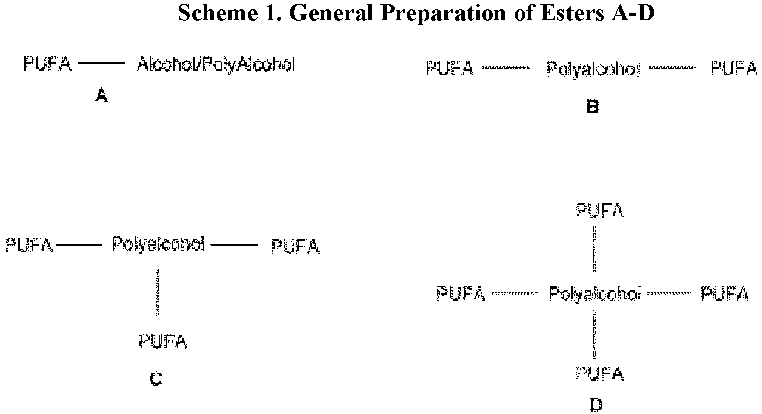

**General Procedure for Compound** A. Thionyl chloride (2 equivalents) is slowly added to a solution of polyunsaturated fatty acid (1 equivalent) in CHC1₃. The reaction mixture is heated to reflux for 1 hr, then it is allowed to cool to room temperature and the solvent is evaporated under reduced pressure to afford the carboxylic acid chloride derivative of the polyunsaturated fatty acid. The carboxylic acid chloride derivative is then dissolved in anhydrous pyridine and the alcohol (1 equivalent) dissolved in pyridine is slowly added (Note that the order of addition is reversed when the alcohol is a polyalcohol). Upon complete addition, the reaction mixture is allowed to stir at room temperature for 24 hr. The solvent is then removed under reduced pressure and the crude product is purified by column chromatography to afford Compound A.

**General Procedure for Compound B**. Thionyl chloride (2 equivalents) is slowly added to a solution of polyunsaturated fatty acid (1 equivalent) in CHC1₃. The reaction mixture is heated to reflux for 1 hr, then it is allowed to cool to room temperature and the solvent is evaporated under reduced pressure to afford the carboxylic acid chloride derivative of the polyunsaturated fatty acid. The carboxylic acid chloride derivative is then dissolved in anhydrous pyridine and the alcohol (Compound A, 1 equivalent) dissolved in pyridine is slowly added. Upon complete addition, the reaction mixture is allowed to stir at room temperature for 24 hr. The solvent is then removed under reduced pressure and the crude product is purified by column chromatography to afford Compound B.

General Procedure for Compound C. Thionyl chloride (2 equivalents) is slowly added to a solution of polyunsaturated fatty acid (1 equivalent) in CHC1₃. The reaction mixture is heated to reflux for 1 hr, then it is allowed to cool to room temperature and the solvent is evaporated under reduced pressure to afford the carboxylic acid chloride derivative of the polyunsaturated fatty acid. The carboxylic acid chloride derivative is then dissolved in anhydrous pyridine and the alcohol (Compound B, 1 equivalent) dissolved in pyridine is slowly added. Upon complete addition, the reaction mixture is allowed to stir at room temperature for 24 hr. The solvent is then removed under reduced pressure and the crude product is purified by column chromatography to afford Compound C.

General Procedure for Compound D. Thionyl chloride (2 equivalents) is slowly added to a solution of polyunsaturated fatty acid (1 equivalent) in CHC1₃. The reaction mixture is heated to reflux for 1 hr, then it is allowed to cool to room temperature and the solvent is evaporated under reduced pressure to afford the carboxylic acid chloride derivative of the polyunsaturated fatty acid. The carboxylic acid chloride derivative (4 equivalents) is then dissolved in anhydrous pyridine and the alcohol (1 equivalent) dissolved in pyridine is slowly added. Upon complete addition, the reaction mixture is allowed to stir at room temperature for 24 hr. The solvent is then removed under reduced pressure and the crude product is purified by column chromatography to afford Compound D.

### Example 1. Effects on Q-less Yeast

Q-less yeast (coq mutants) provide an ideal system to assess in vivo autoxidation of fatty acids. Coenzyme Q (ubiquinone or Q) serves as a small lipophilic antioxidant as well as an electron shuttle in the respiratory chain of the mitochondrial inner membrane. Ten S. cerevisiae genes (COQ1-COQ10) are required for coenzyme Q biosynthesis and function, and the deletion of any results in respiratory deficiency (Tran UC, Clarke CF. Mitochondrion 2007; 7S,S62). It was shown that the coq yeast mutants are exquisitely sensitive to autoxidation products of PUFAs (Do TQ et al, PNAS USA 1996;93:7534-7539; Poon WW, Do TQ, Marbois BN, Clarke CF. Mol. Aspects Med. 1997;18,s121). Although S. cerevisiae do not produce PUFAs (Paltauf F, Daum G. Meth. Enzymol. 1992;209:514-522)**,** they are able to utilize PUFAs when provided exogenously, allowing their content to be manipulated (Paltauf F, Daum G. Meth. Enzymol. 1992;209:514-522)**.** Less than 1% of Q-less (coq2, coq3, and coq5) yeast mutants are viable following a four hour treatment with linolenic acid (Do TQ et al, PNAS USA 1996;93:7534-7539; Poon WW, Do TQ, Marbois BN, Clarke CF. Mol. Aspects Med. 1997;18,s121). In contrast, 70% of wild-type (the parental genetic background is strain W303-1B) cells subjected to this treatment remain viable. The Q-less yeast are also hypersensitive to other PUFAs that readily autoxidize (such as arachidonic acid), but behave the same as the wild-type parental strain to treatment with the monounsaturated oleic acid (Do TQ et al, PNAS USA 1996;93:7534-7539). The hypersensitivity of the Q-less yeast mutants is not a secondary effect of the inability to respire, because con 1 or atp2 mutant yeast (lacking either the bcl complex or the ATP synthase, respectively) show wild-type resistance to PUFA treatment (Do TQ et al, PNAS USA 1996;93:7534-7539; Poon WW, Do TQ, Marbois BN, Clarke CF. Mol. Aspects Med. 1997;18,s121).

A plate dilution assay can be used to assess PUFA sensitivity. This assay is performed by spotting serial five-fold dilutions of aliquots onto YPD plate media. The sensitivity of the different strains is observed by visual inspection of the density of cells in each spot.

Cell types representative of oxidation-related disorders can also be evaluated. For example, such cells are kept in a medium containing either hydrogenated (control) or isotopically modified/stabilized compounds (20 µM) for 72 hrs. The incorporation of PUFAs into cells is monitored by GC. The cells are then treated with paraquat (PQ; 500 µM), a common oxidative stress-generating compound. For survival measurement, cells are counted using haemocytometer and trypan blue exclusion method.

Alternatively, experiments can be performed in which cells are treated with various concentrations of steroids (glucocorticoids, corticoids, etc.) with pre- and post-PUFA treatment in the presence of an oxidative stress-generating compound, such as paraquat. For survival measurement, cells are counted using haemocytometer and trypan blue exclusion method.

### Example 2. Neuroblastoma Cell Evaluation

Dhcr7-deficient Neuro2a neuroblastoma cells were treated with D2-linoleic acid, and non-deuterated linoleic acid ("Lin") was used in a control experiment. The level of *Dhcr7* gene expression was depleted by RNA interference, leading to accumulation of 7-DHC in the cells. 7-DHC is 1-2 orders of magnitude more oxidizeable than linoleic acid, and so can easily initiate lipid peroxidation (See Yin et al., "Free Radical Lipid Peroxidation: Mechanisms and Analysis," Chem Rev. (2011), 111(10), 5944-5972). It was found that non-deuterated Lin (at 20 micromolar level) substantially increased the levels of oxysterols associated with free radical induced oxidation, while D2-Lin at the same concentration did not induce oxidative stress, judging by the low levels of corresponding oxysterols.

### Example 3. Histopathologic Studies

Histopathologic studies can also be conducted for the compounds disclosed herein. Microscopic changes are coded by the most specific topographic and morphologic diagnosis, and the Systematized Nomenclature of Medicine (SNOMED) and the National Toxicology Program's Toxicology Data Management System (TDMS) terminology manuals are used as guidelines. Data are recorded in Labcat^{®} Histopathology module 4.30. A four-step grading system (minimal, mild, moderate, and marked) is used to define gradable changes.

C57BL6 male mice are dosed orally in the diet with PUFAs on Study Days 1 through 14, and are necropsied on Study Day 15. Group 1 can consist of 4 mice and receive nonisotopically modified polyunsaturated substances. Group 2 can consist of 5 mice and receive isotopically modified polyunsaturated substances. On Study Day 8, all mice received intraperitoneal (IP) saline. Complete sets of protocol-specified tissues [liver (3 to 7 sections), lungs with bronchi (2 to 5 lobes), spleen, heart, and kidneys] from all submitted mice are examined histopathologically to detect differences.

### Example 4. Evaluation of Tissue-specific Isotope Incorporation

Tissue specific isotope incorporate can also be determined. WT mice are housed at 12 animals (males separate from females) per cage and fed for 90 days ad libitum (typically, 5-6 g/day) on the AIN 93 diet, as pellets, with 6% total fat. Approximately 10% of that total fat is made up of isotopically modified compounds or non-modified compounds (control group). The animals are sacrificed, various organs (such as eyes) are harvested and stored at low temperature prior to analysis without the use of preservation agents. Lipid fractions are separated, pre-treated and analyzed by LC-MS according to standard protocols. *See* Shchepinov et al. Toxicology Letters 207 (2011) 97-103. Additionally, experiments can utilize transgenic mice whose genome comprises a homozygous disruption of a Vav2 and/or Vav3 gene but not a disruption in a Vavl gene as a model for evaluating an optic neuropathy such as glaucoma.

### Example 5: Model for Testing Incorporation into Eyes

Isotope ratio Mass-spectrometry can be used to confirm incorporation of test compounds into the phospholipid membranes of various tissues, such as eyes and specific components thereof. When delivering test compounds through dietary supplementation, incorporation into eyes and specific components can be monitored using an isotope ratio mass-spectrometry technique that will allow for measurement of the total increase in deuterium composition in lipid membranes, thus reporting on incorporation of test compounds, and any other PUFA derived from these compounds. Using this method, a substantial uptake of test compounds into eyes and specific components can be detected. For example, mice are supplemented with test compounds (0.01, 0.1, 1.0, 10.0, and 100 mg/kg of) or non-modified compounds (0.01, 0.1, 1.0, 10.0, and 100 mg/kg) as the only source of such compounds for 6 days, exposed acutely to a known oxidant or saline vehicle and continued on the same diet for an additional 6 days. The animals are sacrificed and eyes are collected, compartmentalized as desired, and converted into homogenate samples from control mice and test compound-treated mice for analysis of isotope content.

### Example 6: Efficacy Against Glaucoma

Glaucoma cells from human donor eyes are cultured according to known methods and exposed to D-PUFA (0.01, 0.1, 1.0, 10.0, 100, and 1000 µM D2-LA, D4-ALA, and 1:1 combinations of both D2-LA and D4-ALA) or H-PUFA (0.01, 0.1, 1.0, 10.0, 100, and 1000 µM LA, ALA, and 1:1 combinations of both LA and ALA). At several time points (24, 28, and 72 hours), digital images are taken and analyzed with respect to lipofuscin and pigmentation according to known methods. The deuterated polyunsaturated fatty acid or ester thereof is expected to increase the growth of retinal ganglion cells.

C57BL/6 female mice aged 3 months (n=20) are used as the normal model. DBA/2J female mice aged 8 months (n=20) are used as the glaucoma mouse model. One eye of each animal is treated and the other eye serves as a control (for both the normal and glaucoma models). DBA/2J mice spontaneously develop essential iris atrophy, pigment dispersion, and glaucomatous changes. The intraocular pressure (IOP) becomes elevated in most mice by the age of 9 months, which can be followed by ganglion cell loss, optic nerve atrophy and optic nerve cupping. As glaucoma develops, ganglion cell loss and mild cupping of the optic nerve are present in some animals by 11 months and in the majority of mice by the age of 22 months. T

Accordingly, D-PUFA (0.01, 0.1, 1.0, 10.0, and 100 mg/kg D2-LA, D4-ALA, and 1:1 combinations of both D2-LA and D4-ALA) or H-PUFA (0.01, 0.1, 1.0, 10.0, and 100 mg/kg LA, ALA, and 1:1 combinations of both LA and ALA) are daily administered to mice having glaucoma. Over a twenty week period, the rats are examined for IPO, retinal ganglion cells, and other indicators of glaucoma condition. At the end of the trial, the rats are euthanized, their eyes are dissected, and IPO, retinal ganglion cells, and other indicators of glaucoma condition are further examined. The deuterated polyunsaturated fatty acid or ester thereof reduces IPO, increases the growth of retinal ganglion cells, and alleviates the conditions of glaucoma.

### Example 7: Efficacy against Non-hereditary Optic Neuropathy

Mice having non-hereditary optic neuropathy are raised on a diet of D-PUFA (0.01, 0.1, 1.0, 10.0, and 100 mg/kg D2-LA, D4-ALA, and 1:1 combinations of both D2-LA and D4-ALA) or H-PUFA (0.01, 0.1, 1.0, 10.0, and 100 mg/kg LA, ALA, and 1:1 combinations of both LA and ALA). After 1, 3, 5, and 7 weeks, mice are sacrificed, their eyes are dissected, and the retinas and eyecups are pooled and homogenated with ethanol. The homogenate is centrifuged, the supernatant is drawn off, and the supernatant is analyzed for A2E-lipofuscin by HPLC. The deuterated polyunsaturated fatty acid or ester thereof expected to reduce inflammation of the optic nerve, reduce the loss of myelin.

In mice having non-hereditary optic neuropathy, deuterated polyunsaturated fatty acid or ester thereof such as D-PUFA (0.01, 0.1, 1.0, 10.0, and 100 mg/kg D2-LA, D4-ALA, and 1:1 combinations of both D2-LA and D4-ALA) or H-PUFA (0.01, 0.1, 1.0, 10.0, and 100 mg/kg LA, ALA, and 1:1 combinations of both LA and ALA) are administered to the ocular or adnexal tissue. After 1, 3, 5, and 7 weeks, mice are sacrificed, their eyes are dissected, and the retinas and eyecups are pooled and homogenated with ethanol. The homogenate is centrifuged, the supernatant is drawn off, and the supernatant is analyzed for myelin cell count and other indicators of optic neuropathy. The deuterated polyunsaturated fatty acid or ester thereof reduces inflammation of the optic nerve and reduces the loss of myelin.

### Example 8: Efficacy against Steroid induced oxidative stress or steroid induced oxidation related disorder

Mice having Steroid induced oxidative stress or steroid induced oxidation related disorder are raised on a diet of deuterated polyunsaturated fatty acid or ester thereof such as D-PUFA (0.01, 0.1, 1.0, 10.0, and 100 mg/kg D2-LA, D4-ALA, and 1:1 combinations of both D2-LA and D4-ALA) or H-PUFA (0.01, 0.1, 1.0, 10.0, and 100 mg/kg LA, ALA, and 1:1 combinations of both LA and ALA). After 1, 3, 5, and 7 weeks, mice are sacrificed, their eyes are dissected, and the retinas and eyecups are pooled and homogenated with ethanol. The homogenate is centrifuged, the supernatant is drawn off, and the supernatant is analyzed for A2E-lipofuscin by HPLC. The deuterated polyunsaturated fatty acid or ester thereof reduces the loss of myelin and reduces the oxidative stress in the eye tissue.

In mice having Steroid induced oxidative stress or steroid induced oxidation related disorder, deuterated polyunsaturated fatty acid or ester thereof such as D-PUFA (0.01, 0.1, 1.0, 10.0, and 100 mg/kg D2-LA, D4-ALA, and 1:1 combinations of both D2-LA and D4-ALA) or H-PUFA (0.01, 0.1, 1.0, 10.0, and 100 mg/kg LA, ALA, and 1:1 combinations of both LA and ALA) are administered to the ocular or adnexal tissue of the mice. After 1, 3, 5, and 7 weeks, mice are sacrificed, their eyes are dissected, and the retinas and eyecups are pooled and homogenated with ethanol. The homogenate is centrifuged, the supernatant is drawn off, and the supernatant is analyzed for A2E-lipofuscin by HPLC. The deuterated polyunsaturated fatty acid or ester thereof reduces the loss of myelin and reduces the oxidative stress in the eye tissue.

### Example 9: Treating Patients Having Glaucoma

Glaucoma patients are treated with deuterated polyunsaturated fatty acid or ester thereof, including D-PUFA (0.01, 0.1, 1.0, 10.0, and 100 mg/kg D2-LA, D4-ALA, and 1:1 combinations of both D2-LA and D4-ALA) or H-PUFA (0.01, 0.1, 1.0, 10.0, and 100 mg/kg LA, ALA, and 1:1 combinations of both LA and ALA). After 1, 3, 5, and 7 weeks, the patients are examined for iris atrophy, pigment dispersion, glaucomatous changes, intraocular pressure change, and ganglion cell count. The deuterated polyunsaturated fatty acid or ester thereof is expected to reduce the intraocular pressure change, increase ganglion cell counts, and improve other indicators for glaucoma.

### Example 10: Treating Patients Having Non-hereditary Optic Neuropathy

Patients having non-hereditary optic neuropathy are treated with deuterated polyunsaturated fatty acid or ester thereof, including D-PUFA (0.01, 0.1, 1.0, 10.0, and 100 mg/kg D2-LA, D4-ALA, and 1:1 combinations of both D2-LA and D4-ALA) or H-PUFA (0.01, 0.1, 1.0, 10.0, and 100 mg/kg LA, ALA, and 1:1 combinations of both LA and ALA). After 1, 3, 5, and 7 weeks, the patients are examined for iris atrophy, pigment dispersion, glaucomatous changes, intraocular pressure change, for myelin cell count and other indicators of optic neuropathy. The deuterated polyunsaturated fatty acid or ester thereof is expected to reduce the intraocular pressure change, increase myelin cell counts, and improve the conditions of optic neuropathy.

### Example 11: Treating Patients Having Steroid induced oxidative stress or steroid induced oxidation related disorder

Patients having Steroid induced oxidative stress or steroid induced oxidation related disorder are treated with deuterated polyunsaturated fatty acid or ester thereof, including D-PUFA (0.01, 0.1, 1.0, 10.0, and 100 mg/kg D2-LA, D4-ALA, and 1:1 combinations of both D2-LA and D4-ALA) or H-PUFA (0.01, 0.1, 1.0, 10.0, and 100 mg/kg LA, ALA, and 1:1 combinations of both LA and ALA). After 1, 3, 5, and 7 weeks, the patients are examined for iris atrophy, pigment dispersion, glaucomatous changes, intraocular pressure change, and other indicators for oxidative srtess. The deuterated polyunsaturated fatty acid or ester thereof is expected to reduce the optic oxidative stress.

While the invention has been described with reference to the specific embodiments thereof, it should be understood by those skilled in the art that various changes may be made and equivalents may be substituted without departing from the true spirit and scope of the invention. This includes embodiments which do not provide all of the benefits and features set forth herein. In addition, many modifications may be made to adapt a particular situation, material, composition of matter, process, process step or steps, to the objective, spirit and scope of the present invention. All such modifications are intended to be within the scope of the claims appended hereto. Accordingly, the scope of the invention is defined only by reference to the appended claims.

### LIST OF EMBODIMENTS:

Embodiment 1. A method of treating glaucoma, a non-hereditary optic neuropathy, steroid-induced oxidative stress, or a steroid-induced oxidation-related disorder, comprising:
   identifying a patient having glaucoma, a non-hereditary optic neuropathy, steroid-induced oxidative stress, or a steroid-induced oxidation-related disorders and in need of treatment;
   repeatedly administering a polyunsaturated substance to the patient or the patient's tissue, wherein the polyunsaturated substance is chemically modified such that one or more bonds is stabilized against oxidation;
   wherein following said administration, the polyunsaturated substance or a polyunsaturated metabolite thereof comprising said one or more chemical modifications is incorporated into the patient's body or the patient's tissue.
Embodiment 2. The method of Embodiment 1, wherein the polyunsaturated substance is a fatty acid, a fatty acid mimetic, or a fatty acid pro-drug.
Embodiment 3. The method of any one of Embodiments 1-2, wherein the fatty acid, fatty acid mimetic, or fatty acid pro-drug is stabilized at one or more bis-allylic positions.
Embodiment 4. The method of any one of Embodiments 1-3, wherein the stabilization comprises at least one ¹³C atom or at least one deuterium atom at a bis-allylic position, wherein the at least one ¹³C atom or the at least one deuterium atom is present at a level significantly above the naturally-occurring abundance level of said isotope.
Embodiment 5. The method of any one of Embodiments 1-4, wherein the stabilized fatty acid, fatty acid mimetic, or fatty acid pro-drug comprise between about 10% and 50% of the total amount of fatty acids, fatty acid mimetics, or fatty acid pro-drugs administered to the patient.
Embodiment 6. The method of any one of Embodiments 1-4, wherein the isotopically stabilized fatty acid, fatty acid mimetic, or fatty acid pro-drug comprise between about 10% and 30% of the total amount of fatty acids, fatty acid mimetics, or fatty acid pro-drugs administered to the patient.
Embodiment 7. The method of any one of Embodiments 1-4, wherein the isotopically stabilized fatty acid, fatty acid mimetic, or fatty acid pro-drug comprise about 20% or more of the total amount of fatty acids, fatty acid mimetics, or fatty acid pro-drugs administered to the patient.
Embodiment 8. The method of any one of Embodiments 1-4, wherein a cell or tissue of the patient maintains a sufficient concentration of the fatty acid, fatty acid mimetic, or fatty acid pro-drug to prevent autooxidation of the naturally occurring polyunsaturated fatty acid, mimetic, or ester pro-drug.
Embodiment 9. The method of any one of Embodiments 1-4, wherein the polyunsaturated substance is an omega-3 fatty acid, fatty acid mimetic, or fatty acid pro-drug, or an omega-6 fatty acid, fatty acid mimetic, or fatty acid pro-drug.
Embodiment 10. The method of any one of Embodiments 1-9, wherein the polyunsaturated substance is selected from the group consisting of 11,11-D2-linolenic acid, 14,14-D2-linolenic acid, 11,11,14,14-D4-linolenic acid, 11,11-D2-linoleic acid, 14,14-D2-linoleic acid, 11,11,14,14-D4-linoleic acid, 11-D-linolenic acid, 14-D-linolenic acid, 11,14-D2-linolenic acid, 11-D-linoleic acid, 14-D-linoleic acid, and 11,14-D2-linoleic acid.
Embodiment 11. The method of any one of Embodiments 1-9, wherein the polyunsaturated substance is further stabilized at a pro-bis-allylic position.
Embodiment 12. The method of any one of Embodiments 1-4, wherein the fatty acid pro-drug is an ester.
Embodiment 13. The method of any one of Embodiments 1-12, wherein the ester is a triglyceride, diglyceride, or monoglyceride.
Embodiment 14. The method of any one of Embodiments 1-13 further comprising coadministering an antioxidant.
Embodiment 15. The method of Embodiment 14, wherein the antioxidant is Coenzyme Q, idebenone, mitoquinone, mitoquinol, vitamin C, or vitamin E.
Embodiment 16. The method of any one of Embodiments 1-15-, wherein the polyunsaturated substance is further chemically modified at one or more bis-allylic positions.
Embodiment 17. The method of any one of Embodiments 1-16, wherein the stabilized fatty acid, fatty acid mimetic, or fatty acid pro-drug comprise between about 10% and 50% of the total amount of fatty acids, fatty acid mimetics, or fatty acid pro-drugs administered to the patient.
Embodiment 18. The method of any one of Embodiments 1-17, wherein the isotopically stabilized fatty acid, fatty acid mimetic, or fatty acid pro-drug comprise between about 10% and 30% of the total amount of fatty acids, fatty acid mimetics, or fatty acid pro-drugs administered to the patient.
Embodiment 19. The method of any one of Embodiments 1-18, wherein the isotopically stabilized fatty acid, fatty acid mimetic, or fatty acid pro-drug comprise about 20% or more of the total amount of fatty acids, fatty acid mimetics, or fatty acid pro-drugs administered to the patient.
Embodiment 20. The method of any one of Embodiments 1-19, wherein a cell or tissue of the patient maintains a sufficient concentration of polyunsaturated substance to inhibit autooxidation.
Embodiment 21. The method of any one of Embodiments 1-20, wherein the polyunsaturated substance is an omega-3 fatty acid, an omega-3 fatty acid mimetic, or an omega-3 fatty acid pro-drug, or an omega-6 fatty acid, omega-6 fatty acid mimetic, or omega-6 fatty acid pro-drug.
Embodiment 22. The method of any one of Embodiments 1-21, wherein the polyunsaturated substance is selected from the group consisting of 9,10,12,13,15,16-D6-linolenic acid, 9,10,11,12,13,15,16-D7-linolenic acid, 9,10,11,11,12,13,15,16-D8-linolenic acid, 9,10,12,13,14,14,15,16-D8-linolenic acid, 9,10,12,13,14,15,16-D7-linolenic acid, 9,10,11,11,12,13,14,14,15,16-D10-linolenic acid, 9,10,11,12,13,14,15,16-D8-linolenic acid, 9,10,12,13-D4-linoleic acid, 9,10,11,12,13 -D5-linoleic acid, 9,10,11,11,12,13 -D6-linoleic acid, or alkyl ester pro-drug thereof.
Embodiment 23. The method of any one of Embodiments 1-22, wherein the polyunsaturated substance is 9,10,12,13-D4-linoleic acid or an alkyl ester pro-drug thereof.
Embodiment 24. The method of any one of Embodiments 1-23, wherein the polyunsaturated substance is further chemically modified at one or more bis-allylic positions, wherein the chemical modification comprises at least one ¹³C atom at the one or more bis-allylic positions or at least one deuterium atom at the one or more bis-allylic positions, and wherein the at least one 13C atom or the at least one deuterium atom is present at a level significantly above the naturally-occurring abundance level of said isotope.
Embodiment 25. The method of any one of Embodiments 1-24, wherein the polyunsaturated substance is further chemically modified at one or more bis-allylic positions, wherein the chemical modification comprises at least one or more deuterium atoms at the one or more bis-allylic positions, and wherein the at least one or more deuterium atoms is present at a level significantly above the naturally-occurring abundance level of said isotope.
Embodiment 26. The method of any one of Embodiments 1-25, wherein the polyunsaturated substance is further chemically modified at one or more bis-allylic positions, and wherein the chemical modification comprises at two deuterium atoms at one or more bis-allylic position, and wherein the two deuterium atoms are present at a level significantly above the naturally-occurring abundance level of said isotope.
Embodiment 27. The method of any one of Embodiments 1-26, wherein the polyunsaturated substance is a fatty acid pro-drug ester.
Embodiment 28. The method of any one of Embodiments 1-27, wherein the polyunsaturated substance is a fatty acid pro-drug ester and the ester is a triglyceride, diglyceride, or monoglyceride.
Embodiment 29. The method of any one of Embodiments 1-28, wherein the polyunsaturated substance is a fatty acid pro-drug ester and the ester is an alkyl ester.
Embodiment 30. The method of any one of Embodiments 1-29, wherein the polyunsaturated substance is a fatty acid pro-drug ester and the ester is an ethyl ester.
Embodiment 31. The method of any one of Embodiments 1-30 further comprising co-administering an antioxidant.
Embodiment 32. The method of any one of Embodiments 1-31, further comprising co-administering an antioxidant, and wherein the antioxidant is Coenzyme Q, idebenone, mitoquinone, mitoquinol, vitamin C, or vitamin E.
Embodiment 33. The method of any one of Embodiments 1-32, wherein the patient is a mammal.
Embodiment 34. The method of Embodiment 33, wherein the patient is a human.
Embodiment 35. The method of Embodiment 34, wherein the patient is a male.
Embodiment 36. The method of Embodiment 34, wherein the patient is a female.
Embodiment 37. The method of any one of Embodiments 1-36, wherein the patient is classified as having a non-hereditary optic neuropathy.
Embodiment 38. The method of Embodiment 37, wherein the polyunsaturated substance is repeatedly administered to the patient beginning prior to surgical intervention or the administration of a second therapeutic to treat the optic neuropathy.
Embodiment 39. The method of Embodiment 37, wherein the polyunsaturated substance is repeatedly administered to the patient beginning concurrently with surgical intervention or the administration of a second therapeutic to treat the optic neuropathy.
Embodiment 40. The method of Embodiment 37, wherein the polyunsaturated substance is repeatedly administered to the patient beginning after surgical intervention or the administration of a second therapeutic to treat the optic neuropathy.
Embodiment 41. The method of Embodiment 37, wherein the non-hereditary optic neuropathy is glaucoma.
Embodiment 42. The method of any one of Embodiments 1-41, wherein the patient is classified as having glaucoma.
Embodiment 43. The method of Embodiment 42, wherein the glaucoma is primary glaucoma, developmental glaucoma, secondary glaucoma, or absolute glaucoma.
Embodiment 44. The method of Embodiment 43, wherein the glaucoma is primary angle closure glaucoma, primary open-angle glaucoma, pigmentary glaucoma, or exfoliation glaucoma.
Embodiment 45. The method of Embodiment 43, wherein the glaucoma is primary congenital glaucoma, infantile glaucoma, or inheritable glaucoma.
Embodiment 46. The method of Embodiment 43, wherein the glaucoma is an inflammatory glaucoma, a phacogenic glaucoma, a glaucoma secondary to intraocular hemorrhage, a traumatic glaucoma, a neovascular glaucoma, a drug-induced glaucoma, or a glaucoma of miscellaneous origin.
Embodiment 47. The method of any one of Embodiments 1-46, wherein the patient is classified as having steroid-induced oxidative stress.
Embodiment 48. The method of Embodiment 47, wherein the polyunsaturated substance is repeatedly administered to the patient beginning prior to the administration of a steroid for treatment of a disease or symptom.
Embodiment 49. The method of Embodiment 48, wherein the polyunsaturated substance is repeatedly administered to the patient beginning concurrently with the administration of a steroid for treatment of a disease or symptom.
Embodiment 50. The method of Embodiment 49, wherein the polyunsaturated substance is repeatedly administered to the patient beginning after the administration of a steroid for treatment of a disease or symptom.
Embodiment 51. The method of any one of Embodiments 46-50, wherein the polyunsaturated substance treats, reduces, or inhibits cataracts or cataract formation.
Embodiment 52. The method of any one of Embodiments 1-36 and 46-50, wherein the patient is classified as having a steroid-induced oxidation-related disorder.
Embodiment 53. The method of Embodiment 52, wherein the steroid-induced oxidation disorder is a cataract.
Embodiment 54. A polyunsaturated compound for use in treating glaucoma, a non-hereditary optic neuropathy, steroid-induced oxidative stress, or a steroid-induced oxidation-related disorder, according to the method of any one of Embodiments 1-53.
Embodiment 55. Use of a polyunsaturated compound in preparation of a medicament for treating glaucoma, a non-hereditary optic neuropathy, steroid-induced oxidative stress, or a steroid-induced oxidation-related disorder, according to the method of any one of Embodiments 1-53.
Embodiment 56. Use of a polyunsaturated compound for treating glaucoma, a non-hereditary optic neuropathy, steroid-induced oxidative stress, or a steroid-induced oxidation-related disorder, according to the method set forth in any one of Embodiments 1-53.

## Claims

1. A composition comprising a polyunsaturated fatty acid, or ester thereof, isotopically modified at one or more positions to comprise at least one deuterium atom or at least one ¹³C atom for use in a method of treating optic neuropathy, wherein said method comprises:
repeatedly administering said composition to a patient or a tissue of said patient wherein said patient has optic neuropathy due to Leber's hereditary optic neuropathy (LHON); dominant optic atrophy; or Behr's syndrome;
wherein following said administration, the isotopically modified polyunsaturated substance is incorporated into the patient's body or the patient's tissue thereby treating said optic neuropathy.

2. The composition for use of Claim 1, wherein the polyunsaturated fatty acid, or ester thereof, is isotopically modified at one or more bis-allylic positions.

3. The composition for use of Claim 1 or Claim 2, wherein the deuterium atom or the ¹³C atom is present at a level significantly above the naturally-occurring abundance level of said isotope.

4. The composition for use of any one of Claims 1 to 3, wherein the isotopically modified polyunsaturated fatty acid, or ester thereof, is at least about 5% by weight of the total amount of fatty acids or fatty acid esters delivered to the patient.

5. The composition for use of any one of Claims 1 to 4, wherein the polyunsaturated fatty acid, or ester thereof, is an omega-3 fatty acid, an omega-3 fatty acid ester, an omega-6 fatty acid, or an omega-6 fatty acid ester.

6. The composition for use of any one of Claims 1 to 5, wherein the polyunsaturated fatty acid, or ester thereof, is a deuterated linoleic acid, a deuterated linolenic acid, or any ester thereof.

7. The composition for use of Claim 6, wherein the polyunsaturated fatty acid, or ester thereof, is selected from the group consisting of 11,11-D2-linolenic acid, 14,14-D2-linolenic acid, 11,11,14,14-D4-linolenic acid, 11,11-D2-linoleic acid, 14,14-D2-linoleic acid, 11,11,14,14-D4-linoleic acid, 11-D-linolenic acid, 14-D-linolenic acid, 11,14-D2-linolenic acid, 11-D-linoleic acid, 14-D-linoleic acid, and 11,14-D2-linoleic acid, and any esters thereof.

8. The composition for use of any one of Claims 1 to 5, wherein the polyunsaturated fatty acid, or ester thereof, is selected from the group consisting of deuterated arachidonic acid, deuterated eicosapentaenoic acid, deuterated docosahexaenoic acid, and any esters thereof.

9. The composition for use of any one of Claims 1 to 5, wherein the polyunsaturated fatty acid, or ester thereof, is 9,10,12,13,15,16-D6-linolenic acid, 9,10,11,12,13,15,16-D7-linolenic acid, 9,10,11,11,12,13,15,16-D8-linolenic acid, 9,10,12,13,14,14,15,16-D8-linolenic acid, 9,10,12,13,14,15,16-D7-linolenic acid, 9,10,11,11,12,13,14,14,15,16-D10-linolenic acid, 9,10,11,12,13,14,15,16-D8-linolenic acid, 9,10,12,13-D4-linoleic acid, 9,10,11,12,13-D5-linoleic acid, 9,10,11,11,12,13-D6-linoleic acid, or ester thereof.

10. The composition for use of any one of Claims 1 to 9, wherein the polyunsaturated fatty acid, or ester thereof, is further isotopically modified at a pro-bis-allylic position.

11. The composition for use of any one of Claims 1 to 10, wherein the ester is a triglyceride, diglyceride, monoglyceride, or an alkyl ester.

12. The composition for use of Claim 11, wherein the ester is an ethyl ester.

13. The composition for use of any one of Claims 1 to 12, further comprising co-administering at least one an antioxidant.

14. The composition for use of Claim 13, wherein the antioxidant is Coenzyme Q, idebenone, mitoquinone, mitoquinol, vitamin C, or vitamin E, or combinations thereof.

15. The composition for use of Claim 14, wherein the polyunsaturated fatty acid, or ester thereof, is repeatedly administered to the patient beginning prior to, concurrently with, or after surgical intervention or the administration of a second therapeutic to treat the optic neuropathy.
